# EUROPÄISCHE PATENTSCHRIFT

(11) **EP 2 238 149 B1**
(45) Veröffentlichungstag und Bekanntmachung des Hinweises auf die Patenterteilung: **18.03.2015**
(21) Anmeldenummer: 08867523.6
(22) Anmeldetag: 23.12.2008
(51) Int. Cl.: C07J 53/00, A61K 31/585, A61P 5/34

(54) **19-NOR-STEROIDDERIVATE MIT EINER 15ALPHA, 16ALPHA-METHYLENGRUPPE UND EINEM GESÄTTIGTEN 17,17-SPIROLACTONRING, DEREN VERWENDUNG SOWIE DIESE DERIVATE ENTHALTENDE ARZNEIMITTEL**
19-NOR-STEROID DERIVATIVES WITH A 15ALPHA, 16ALPHA-METHYLENE GROUP AND A SATURATED 17,17-SPIROLACTONE RING, USE THEREOF, AND MEDICAMENTS CONTAINING SAID DERIVATIVES
DÉRIVÉS DE 19-NOR-STÉROÏDE PRÉSENTANT UN GROUPE 15ALPHA, 16ALPHA MÉTHYLÈNE ET UN CYCLE 17,17-SPIROLACTONIQUE SATURÉ, LEUR UTILISATION ET MÉDICAMENTS CONTENANT CES DÉRIVÉS

(30) Priorität: 29.12.2007 DE 102007063495
(43) Veröffentlichungstag der Anmeldung: 13.10.2010
(73) Patentinhaber: Bayer Intellectual Property GmbH, 40789 Monheim (DE)
(72) Erfinder: KLAR, Ulrich, 13503 Berlin (DE); KUHNKE, Joachim, 14482 Potsdam (DE); BOHLMANN, Rolf, 14055 Berlin (DE); HÜBNER, Jan, 10317 Berlin (DE); RING, Sven, 07749 Jena (DE); FRENZEL, Thomas, 65719 Hofheim (DE); MENGES, Frederik, 69198 Schriesheim (DE); BORDEN, Steffen, 13355 Berlin (DE); MUHN, Hans-Peter, 13465 Berlin (DE); PRELLE, Katja, 13465 Berlin (DE)
(74) Vertreter: BIP Patents
(86) Internationale Anmeldenummer: PCT/EP2008/011165
(87) Internationale Veröffentlichungsnummer: WO 2009/083272

(56) Entgegenhaltungen:
- EP-A- 0 150 157
- WO-A-2006/072467
- KANDEMIRLI FATMA ET AL: "Investigation of structure-activity relationship on 17-spirolactone derivatives: the electronic-topological approach" FARMACO, SOCIETA CHIMICA ITALIANA, PAVIA, IT, Bd. 57, Nr. 7, 1. Juli 2002 (2002-07-01), Seiten 601-607, XP002418597 ISSN: 0014-827X

## Beschreibung

Die Erfindung betrifft 15α,16α-Methylen-17-hydroxy-19-nor-17-pregna-4-en-3-on-21-carbonsäure γ-Lacton-Derivate mit gestagener Wirkung, deren Verwendung sowie die Derivate enthaltende Arzneimittel, beispielsweise zur Behandlung von prä-, peri- und postmenopausalen sowie von prämenstruellen Beschwerden.

Aus der Literatur sind Verbindungen mit gestagener, antimineralcorticoider, antiandrogener oder antiestrogener Wirkung auf Basis eines Steroidgerüstes bekannt, welche beispielsweise von 19-Nor-androst-4-en-3-on oder einem Derivat davon abgeleitet sind (die Nummerierung des Steroidgerüstes ist beispielsweise Fresenius/Görlitzer 3.Aufl. 1991 "Organisch-chemische Nomenklatur" S. 60 ff. zu entnehmen).

So offenbart WO 2006072467 A1 die als Gestagen wirkende Verbindung 6β,7β-15β,16β-Dimethylen-3-oxo-17-pregn-4-en-21,17β-carbolacton (Drospirenon), welche beispielsweise in einem oralen Kontrazeptivum sowie einem Präparat zur Behandlung postmenopausaler Beschwerden verwendet wurde. Aufgrund seiner vergleichsweise geringen Affinität zum Gestagenrezeptor und seiner vergleichsweise hohen Ovulationshemmdosis ist Drospirenon in dem Kontrazeptivum jedoch in der relativ hohen täglichen Dosis von 3 mg enthalten. Drospirenon zeichnet sich darüber hinaus auch dadurch aus, dass es zusätzlich zur gestagenen Wirkung über aldosteronantagonistische (antimineralcorticoide) sowie antiandrogene Wirkung verfügt. Diese beiden Eigenschaften machen Drospirenon in seinem pharmakologischen Profil dem natürlichen Gestagen Progesteron sehr ähnlich, welches aber anders als Drospirenon nicht ausreichend oral bioverfügbar ist. Um die zu verabreichende Dosis zu senken, werden in WO 2006072467 A1 weiter ein 18-Methyl-19-nor-17-pregn-4-en-21,17-carbolacton sowie diese enthaltende pharmazeutische Präparate vorgeschlagen, welche über eine höhere gestagene Potenz als Drospirenon verfügen.

Daneben offenbart beispielsweise US-A 3,705,179 Steroide, welche eine antiandrogene Aktivität aufweisen und sich zur Behandlung von Krankheiten eignen, die im Zusammenhang mit Androgenen stehen.

Die Aufgabe der vorliegenden Erfindung ist es, Verbindungen zur Verfügung zu stellen, die über eine starke Bindung, und zwar vorzugsweise über eine stärkere als das Drospirenon, an den Gestagenrezeptor verfügen. Außerdem sollen die Verbindungen bevorzugt auch eine antimineralcorticoide Wirkung sowie eine im Hinblick auf den Androgenrezeptor neutrale bis leicht androgene Wirkung aufweisen. Ein weiteres wesentliches Ziel der vorliegenden Erfindung besteht auch darin, ein ausgewogenes Wirkungsprofil hinsichtlich der gestagenen Wirkung zur antimineralcorticoiden Wirkung dergestalt zu erreichen, dass das Verhältnis der gestagenen zur antimineralcorticoiden Wirkung geringer ist als bei Drospirenon.

Diese Aufgabe wird durch die erfindungsgemäßen 15α,16α-Methylen-17-hydroxy-19-nor-17-pregna-4-en-3-on-21-carbonsäure γ-Lacton-Derivate gemäß Anspruch 1, die Verwendung der erfindungsgemäßen Derivate gemäß Anspruch 12 sowie ein mindestens ein erfindungsgemäßes Derivat enthaltendes Arzneimittel gemäß Anspruch 14 gelöst. Vorteilhafte Ausführungsformen der Erfindung sind in den Unteransprüchen angegeben.

Die vorliegende Erfindung beschreibt die neuen 15,16-Methylen-17-hydroxy-19-nor-17-pregna-4,20(Z)-dien-3-on-21-carbonsäure γ-Lacton-Derivate der allgemeinen Formel I, 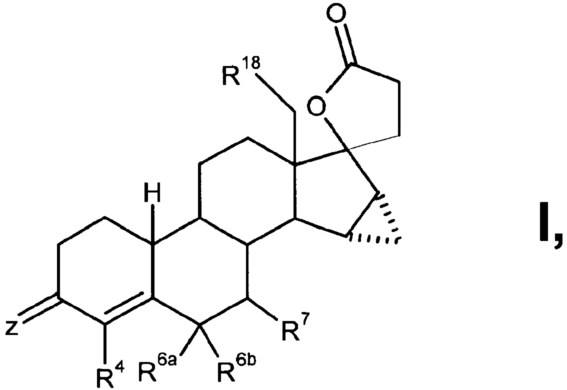 worin
Z Sauerstoff, zwei Wasserstoffatome, eine Gruppe =NOR¹ oder =NNHSO₂R¹,
R¹ Wasserstoff, C₁-C₁₀-Alkyl, Aryl, C₇-C₂₀-Aralkyl,
R⁴ Wasserstoff oder Halogen,
R^{6a}, R^{6b} gleich oder verschieden sind und Wasserstoff, C₁-C₁₀-Alkyl, C₂-C₁₀-Alkenyl, C₂-C₁₀-Alkinyl oder gemeinsam Methylen oder 1,2-Ethandiyl,
R⁷ Wasserstoff, C₁-C₁₀-Alkyl, C₃-C₆-Cycloalkyl, C₂-C₁₀-Alkenyl, C₂-C₁₀-Alkinyl,
R^{6a}, R⁷ gemeinsam eine Bindung, ein Sauerstoff oder eine Methylengruppe,
R¹⁸ Wasserstoff, C₁-C₃-Alkyl,
bedeuten.

Die Reste R^{6a}, R^{6b} und R⁷ sowie der Dreiring können jeweils α- oder β-ständig sein.

Bevorzugt sind Verbindungen der Formel I worin
Z Sauerstoff, eine Gruppe =NOR¹,
R¹ Wasserstoff, C₁-C₆-Alkyl, Aryl, C₇-C₁₂-Aralkyl,
R⁴ Wasserstoff oder Halogen,
R^{6a}, R^{6b} gleich oder verschieden sind und Wasserstoff, C₁-C₆-Alkyl, C₂-C₆-Alkenyl, C₂-C₆-Alkinyl oder gemeinsam Methylen oder 1,2-Ethandiyl,
R⁷ Wasserstoff, C₁-C₆-Alkyl, C₃-C₆-Cycloalkyl, C₂-C₆-Alkenyl, C₂-C₆-Alkinyl,
R^{6a}, R⁷ gemeinsam eine Bindung, oder eine Methylengruppe,
R¹⁸ Wasserstoff, C₁-C₂-Alkyl
bedeuten.

Besonders bevorzugt sind Verbindungen der Formel I worin
Z Sauerstoff, eine Gruppe =NOR¹,
R¹ Wasserstoff, C₁-C₃-Alkyl,
R⁴ Wasserstoff, Chlor oder Brom,
R^{6a}, R^{6b} gleich oder verschieden sind und Wasserstoff, C₁-C₃-Alkyl, C₂-C₄-Alkenyl, oder gemeinsam Methylen oder gemeinsam 1,2-Ethandiyl,
R⁷ Wasserstoff, C₁-C₄-Alkyl, C₃-C₄-Cycloalkyl, C₂-C₄-Alkenyl,
R^{6a}, R⁷ gemeinsam eine Bindung, oder eine Methylengruppe,
R¹⁸ Wasserstoff, Methyl
bedeuten.

Die Nummerierung des C-Gerüstes der erfindungsgemäßen Derivate mit den allgemeinen chemischen Formel I folgt in üblicher Weise der Nummerierung eines Steroidgerüstes, beispielsweise beschrieben in Fresenius, a.a.O. Die Nummerierung der in den Ansprüchen angegebenen Reste entspricht in analoger Weise ihrer Bindungsposition am C-Gerüst der Derivate, soweit dies R⁴, R⁶, R⁷ und R¹⁸ betrifft. So bindet beispielsweise der Rest R⁴ an die C⁴-Position des erfindungsgemäßen Derivats.

Hinsichtlich der zu Z definierten Gruppen binden die Gruppen NOR' und NNHSO₂R' jeweils mit einer Doppelbindung über N an das C-Gerüst des Derivats gemäß =NOR' bzw. =NNH-SO₂R'. OR' in NOR' und NHSO₂R' in NNHSO₂R' können syn- oder antiständig stehen.

Als Alkylgruppen R¹, R^{6a}, R^{6b}, R⁷, R¹⁸, R¹⁹, R²⁰, R^{21a}, R^{21b} und R²² sind gerad- oder verzweigtkettige Alkylgruppen mit 1-10 Kohlenstoffatomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.

Die Alkylgruppen R¹, R^{6a}, R^{6b}, R⁷, R¹⁸, R¹⁹, R²⁰, R^{21a}, R^{21b} und R²² können perfluoriert oder substituiert sein durch 1-5 Halogenatome, Hydroxygruppen, C₁-C₄-Alkoxygruppen, C₆-C₁₂-Arylgruppen (die durch 1-3 Halogenatome substituiert sein können).

Als Alkenylgruppen R^{6a} und R^{6b} sind gerad- oder verzweigtkettige Alkengruppen mit 2-10 Kohlenstoffatomen zu betrachten, wie beispielsweise Vinyl, Propenyl, Butenyl, Pentenyl, Isobutenyl, Isopentenyl.

Als Alkinylgruppen R^{6a} und R^{6b} sind gerad- oder verzweigtkettige Alkingruppen mit 2-10 Kohlenstoffatomen zu betrachten, wie beispielsweise Ethinyl, Propinyl, Butinyl, Pentinyl, Isobutinyl, Isopentinyl.

Die Alkenyl- und Alkinylgruppen R^{6a} und R^{6b} können substituiert sein durch 1-5 Halogenatome, Hydroxygruppen, C₁-C₃-Alkoxygruppen, C₆-C₁₂-Arylgruppen (die durch 1-3 Halogenatome substituiert sein können).

Als Cycloalkylgruppen R⁷ kommen in Betracht Cyclopropyl, Cyclobutyl, Cyclopentyl, Cyclohexyl.

Die Cycloalkylgruppen R⁷ können substituiert sein durch Halogen, OH, O-Alkyl, CO₂H, CO₂-Alkyl, -NH₂, -NO₂, -N₃, -CN, C₁-C₁₀-Alkyl, C₁-C₁₀-Acyl, C₁-C₁₀-Acyloxy-Gruppen.

Als Arylrest R¹, R^{6a}, R^{6b} und R⁷ kommen substituierte und unsubstituierte carbocyclische oder heterocyclische Reste mit einem oder mehreren Heteroatomen wie z.B. Phenyl, Naphthyl, Furyl, Thienyl, Pyridyl, Pyrazolyl, Pyrimidinyl, Oxazolyl, Pyridazinyl, Pyrazinyl, Chinolyl, Thiazolyl, die einfach oder mehrfach substituiert sein können durch Halogen, OH, O-Alkyl, CO₂H, CO₂-Alkyl, -NH₂, -NO₂, -N₃, -CN, C₁-C₁₀-Alkyl, C₁-C₁₀-Acyl, C₁-C₁₀-Acyloxy-Gruppen, in Frage.

Die Aralkylgruppen in R¹ und R⁷ können im Ring bis 14 C-Atome, bevorzugt 6 bis 10 und in der Alkylkette 1 bis 8, bevorzugt 1 bis 4 Atome enthalten. Als Aralkylreste kommen beispielweise in Betracht Benzyl, Phenylethyl, Naphthylmethyl, Naphthylethyl, Furylmethyl, Thienylethyl, Pyridylpropyl. Die Ringe können einfach oder mehrfach substituiert sein durch Halogen, OH, O-Alkyl, CO₂H, CO₂-Alkyl, -NO₂, -N₃, -CN, C₁-C₂₀-Alkyl, C₁-C₂₀-Acyl, C₁-C₂₀-Acyloxy-Gruppen.

### Halogen bedeutet Fluor, Chlor oder Brom.

Die erfindungsgemäßen Derivate können auch in Form von Solvaten, insbesondere von Hydraten vorliegen, wobei die erfindungsgemäßen Verbindungen demgemäß polare Lösungsmittel, insbesondere von Wasser, als Strukturelement des Kristallgitters der erfindungsgemäßen Verbindungen enthalten. Das polare Lösungsmittel, insbesondere Wasser, kann in einem stöchiometrischen oder auch unstöchiometrischen Verhältnis vorliegen. Bei stöchiometrischen Solvaten, Hydraten spricht man auch von Hemi-, (Semi-), Mono-, Sesqui-, Di-, Tri-, Tetra-, Penta-, usw. Solvaten oder Hydraten.

Es wurde gefunden, dass die erfindungsgemäßen Verbindungen bzw. Derivate eine gute gestagene Wirkung aufweisen. Außerdem interagieren einige interessante erfindungsgemäße Verbindungen mit dem Mineralcorticoidrezeptor und sind in der Lage, eine antagonistische Wirkung zu vermitteln. Ferner weisen die erfindungsgemäßen Verbindungen im Hinblick auf den Androgenrezeptor eine neutrale bis leicht androgene Wirkung auf Eine weitere Eigenschaft der Verbindungen besteht darin, dass die Bindungen dieser Verbindungen an den Progesteronrezeptor und an den Mineralcorticoidrezeptor relativ zueinander ausgewogen sind, und zwar dergestalt, dass bei ihnen das Verhältnis der Bindungsfähigkeit zum Progesteronrezeptor zur Bindungsfähigkeit zum Mineralcorticoidrezeptor geringer ist als bei Drospirenon. Somit ist die antimineralcorticoide Wirkung dieser Verbindungen bei gegebener gestagener Wirkung geringer als bei Drospirenon. Wird die Dosierung einer gegebenen erfindungsgemäßen Verbindung aufgrund von deren gestagener Wirkung festgelegt, so ist die antimineralcorticoide Wirkung dieser Verbindung bei dieser Dosierung somit geringer als bei Drospirenon.

Die nachstehend genannten Verbindungen sind erfindungsgemäß bevorzugt:

| | |
|---|---|
| 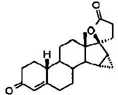 | 17β-Hydroxy-15α,16α-methylen-19-nor-17α-pregna-4-en-3-on-21-carbonsäure γ-Lacton |
| 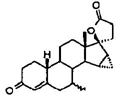 | 17β-Hydroxy-7α-methyl-15α,16α-methylen-19-nor-17α-pregna-4-en-3-on-21-carbonsäure γ-Lacton |
| | 17β-Hydroxy-7β-methyl-15α,16α-methylen-19-nor-17α-pregna-4-en-3-on-21-carbonsäure γ-Lacton |
| 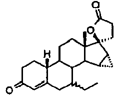 | 17β-Hydroxy-7α-ethyl-15α,16α-methylen-19-nor-17α-pregna-4-en-3-on-21-carbonsäure γ-Lacton |
| | 17β-Hydroxy-7β-ethyl-15α,16α-methylen-19-nor-17α-pregna-4-en-3-on-21-carbonsäure γ-Lacton |
| 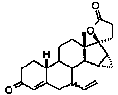 | 17β-Hydroxy-7α-vinyl-15α,16α-methylen-19-nor-17α-pregna-4-en-3-on-21-carbonsäure γ-Lacton |
| | 17β-Hydroxy-7β-vinyl-15α,16α-methylen-19-nor-17α-pregna-4-en-3-on-21-carbonsäure γ-Lacton |
| 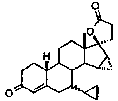 | 17β-Hydroxy-7α-cyclopropyl-15α,16α-methylen-19-nor-17α-pregna-4-en-3-on-21-carbonsäure γ-Lacton |
| | 17β-Hydroxy-7β-cyclopropyl-15α,16α-methylen-19-nor-17α-pregna-4-en-3-on-21-carbonsäure γ-Lacton |
| 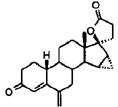 | 17β-Hydroxy-6-methylen-15α,16α-methylen-19-nor-17α-pregna-4-en-3-on-21-carbonsäure γ-Lacton |
| 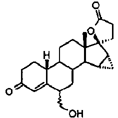 | 17β-Hydroxy-6α-hydroxymethyl-15α,16α-methylen-19-nor-17α-pregna-4-en-3-on-21-carbonsäure γ-Lacton |
| | 17β-Hydroxy-6β-hydroxymethyl-15α,16α-methylen-19-nor-17α-pregna-4-en-3-on-21-carbonsäure γ-Lacton |
| 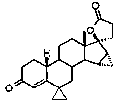 | 6,6-(1,2-Ethandiyl)-17β-hydroxy-15α,16α-methylen-19-nor-17α-pregna-4-en-3-on-21-carbonsäure γ-Lacton |
| 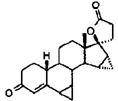 | 17β-Hydroxy-6α,7α,15α,16α-bismethylen-19-nor-17α-pregna-4-en-3-on-21-carbonsäure γ-Lacton |
| | 17β-Hydroxy-6β,7β,15α,16α-bismethylen-19-nor-17α-pregna-4-en-3-on-21-carbonsäure γ-Lacton |
| 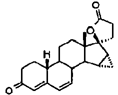 | 17β-Hydroxy-15α,16α-methylen-19-nor-17α-pregna-4,6-dien-3-on-21-carbonsäure γ-Lacton |
| 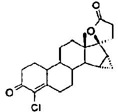 | 4-Chlor-17β-hydroxy-15α,16α-methylen-19-nor-17α-pregna-4-en-3-on-21-carbonsäure γ-Lacton |
| 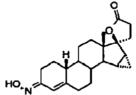 | (E/Z)-3-(Hydroxyimino)-17β-hydroxy-15α,16α-methylen-19-nor-17α-pregna-4-en-21-carbonsäure γ-Lacton |
| 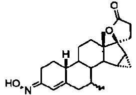 | (E/Z)-3-(Hydroxyimino)-17β-hydroxy-7α-methyl-15α,16α-methylen-19-nor-17α-pregna-4-en-21-carbonsäure γ-Lacton |
| | (E/Z)-3-(Hydroxyimino)-17β-hydroxy-7β-methyl-15α,16α-methylen-19-nor-17α-pregna-4-en-21-carbonsäure γ-Lacton |
| 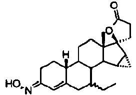 | (E/Z)-3-(Hydroxyimino)-17β-hydroxy-7α-ethyl-15α,16α-methylen-19-nor-17α-pregna-4-en-21-carbonsäure γ-Lacton |
| | (E/Z)-3-(Hydroxyimino)-17β-hydroxy-7β-ethyl-15α,16α-methylen-19-nor-17α-pregna-4-en-21-carbonsäure γ-Lacton |
| 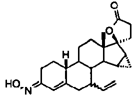 | (E/Z)-3-(Hydroxyimino)-17β-hydroxy-7α-vinyl-15α,16α-methylen-19-nor-17α-pregna-4-en-21-carbonsäure γ-Lacton |
| | (E/Z)-3-(Hydroxyimino)-17β-hydroxy-7β-vinyl-15α,16α-methylen-19-nor-17α-pregna-4-en-21-carbonsäure γ-Lacton |
| 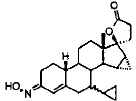 | (E/Z)-3-(Hydroxyimino)-17β-hydroxy-7α-cyclopropyl-15α,16α-methylen-19-nor-17α-pregna-4-en-21-carbonsäure γ-Lacton |
| | (E/Z)-3-(Hydroxyimino)-17β-hydroxy-7β-cyclopropyl-15α,16α-methylen-19-nor-17α-pregna-4-en-21-carbonsäure γ-Lacton |
| 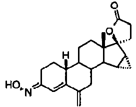 | (E/Z)-3-(Hydroxyimino)-17β-hydroxy-6-methylen-15α,16α-methylen-19-nor-17α-pregna-4-en-21-carbonsäure γ-Lacton |
| 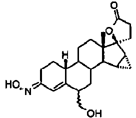 | (E/Z)-3-(Hydroxyimino)-17β-hydroxy-6α-hydroxymethyl-15α,16α-methylen-19-nor-17α-pregna-4-en-21-carbonsäure γ-Lacton |
| | (E/Z)-3-(Hydroxyimino)-17β-hydroxy-6β-hydroxymethyl-15α,16α-methylen-19-nor-17α-pregna-4-en-21-carbonsäure γ-Lacton |
| 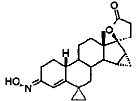 | (E/Z)-3-(Hydroxyimino)-6,6-(1,2-Ethandiyl)-17β-hydroxy-15α,16α-methylen-19-nor-17α-pregna-4-en-21-carbonsäure γ-Lacton |
| 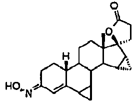 | (E/Z)-3-(Hydroxyimino)-17β-hydroxy-6α,7α,15α,16α-bismethylen-19-nor-17α-pregna-4-en-21-carbonsäure γ-Lacton |
| | (E/Z)-3-(Hydroxyimino)-17β-hydroxy-6β,7β,15α,16α-bismethylen-19-nor-17α-pregna-4-en-21-carbonsäure γ-Lacton |
| 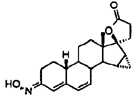 | (E/Z)-3-(Hydroxyimino)-17β-hydroxy-15α,16α-methylen-19-nor-17α-pregna-4,6-dien-21-carbonsäure γ-Lacton |
| 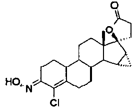 | (E/Z)-3-(Hydroxyimino)-4-Chlor-17β-hydroxy-15α,16α-methylen-19-nor-17α-pregna-4-en-21-carbonsäure γ-Lacton |
| 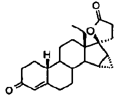 | 17β-Hydroxy-18-methyl-15α,16α-methylen-19-nor-17α-pregna-4-en-3-on-21-carbonsäure γ-Lacton |
| 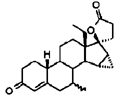 | 17β-Hydroxy-7α-methyl-18-methyl-15α,16α-methylen-19-nor-17α-pregna-4-en-3-on-21-carbonsäure γ-Lacton |
| | 17β-Hydroxy-7β-methyl-18-methyl-15α,16α-methylen-19-nor-17α-pregna-4-en-3-on-21-carbonsäure γ-Lacton |
| 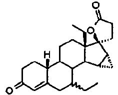 | 17β-Hydroxy-7α-ethyl-18-methyl-15α,16α-methylen-19-nor-17α-pregna-4-en-3-on-21-carbonsäure γ-Lacton |
| | 17β-Hydroxy-7β-ethyl-18-methyl-15α,16α-methylen-19-nor-17α-pregna-4-en-3-on-21-carbonsäure γ-Lacton |
| 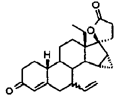 | 17β-Hydroxy-7α-vinyl-18-methyl-15α,16α-methylen-19-nor-17α-pregna-4-en-3-on-21-carbonsäure γ-Lacton |
| | 17β-Hydroxy-7β-vinyl-18-methyl-15α,16α-methylen-19-nor-17α-pregna-4-en-3-on-21-carbonsäure γ-Lacton |
| 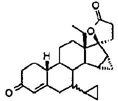 | 17β-Hydroxy-7α-cyclopropyl-18-methyl-15α,16α-methylen-19-nor-17α-pregna-4-en-3-on-21-carbonsäure γ-Lacton |
| | 17β-Hydroxy-7β-cyclopropyl-18-methyl-15α,16α-methylen-19-nor-17α-pregna-4-en-3-on-21-carbonsäure γ-Lacton |
| 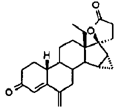 | 17β-Hydroxy-6-methylen-18-methyl-15α,16α-methylen-19-nor-17α-pregna-4-en-3-on-21-carbonsäure γ-Lacton |
| 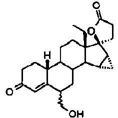 | 17β-Hydroxy-6a-hydroxymethyl-18-methyl-15α,16α-methylen-19-nor-17α-pregna-4-en-3-on-21-carbonsäure γ-Lacton |
| | 17β-Hydroxy-6β-hydroxymethyl-18-methyl-15α,16α-methylen-19-nor-17α-pregna-4-en-3-on-21-carbonsäure γ-Lacton |
| 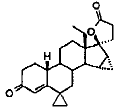 | 6,6-(1,2-Ethandiyl)-17β-hydroxy-18-methyl-15α,16α-methylen-19-nor-17α-pregna-4-en-3-on-21-carbonsäure γ-Lacton |
| 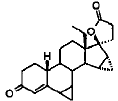 | 17β-Hydroxy-6α,7α,15α,16α-bismethylen-19-nor-17α-pregna-4-en-3-on-21-carbonsäure γ-Lacton |
| | 17β-Hydroxy-6β,7β,15α,16α-bismethylen-19-nor-17α-pregna-4-en-3-on-21-carbonsäure γ-Lacton |
| 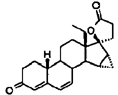 | 17β-Hydroxy-18-methyl-15α,16α-methylen-19-nor-17α-pregna-4,6-dien-3-on-21-carbonsäure γ-Lacton |
| 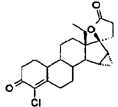 | 4-Chlor-17β-hydroxy-15α,16α-methylen-19-nor-17α-pregna-4-en-3-on-21-carbonsäure γ-Lacton |
| 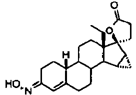 | (E/Z)-3-(Hydroxyimino)-17β-hydroxy-18-methyl-15α,16α-methylen-19-nor-17α-pregna-4-en-21-carbonsäure γ-Lacton |
| 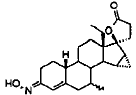 | (E/Z)-3-(Hydroxyimino)-17β-hydroxy-7α-methyl-18-methyl-15α,16α-methylen-19-nor-17α-pregna-4-en-21-carbonsäure γ-Lacton |
| | (E/Z)-3-(Hydroxyimino)-17β-hydroxy-7β-methyl-18-methyl-15α,16α-methylen-19-nor-17α-pregna-4-en-21-carbonsäure γ-Lacton |
| 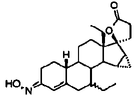 | (E/Z)-3-(Hydroxyimino)-17β-hydroxy-7α-ethyl-18-methyl-15α,16α-methylen-19-nor-17α-pregna-4-en-21-carbonsäure γ-Lacton |
| | (E/Z)-3-(Hydroxyimino)-17β-hydroxy-7β-ethyl-18-methyl-15α,16α-methylen-19-nor-17α-pregna-4-en-21-carbonsäure γ-Lacton |
| 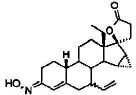 | (E/Z)-3-(Hydroxyimino)-17β-hydroxy-7α-vinyl-18-methyl-15α,16α-methylen-19-nor-17α-pregna-4-en-21-carbonsäure γ-Lacton |
| | (E/Z)-3-(Hydroxyimino)-17β-hydroxy-7β-vinyl-18-methyl-15α,16α-methylen-19-nor-17α-pregna-4-en-21-carbonsäure γ-Lacton |
| 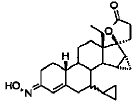 | (E/Z)-3-(Hydroxyimino)-17β-hydroxy-7α-cyclopropyl-18-methyl-15α,16α-methylen-19-nor-17a-pregna-4-en-21-carbonsäure γ-Lacton |
| | (E/Z)-3-(Hydroxyimino)-17β-hydroxy-7β-cyclopropyl-18-methyl-15α,16α-methylen-19-nor-17α-pregna-4-en-21-carbonsäure γ-Lacton |
| 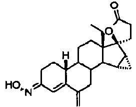 | (E/Z)-3-(Hydroxyimino)-17β-hydroxy-6-methylen-18-methyl-15α,16α-methylen-19-nor-17α-pregna-4-en-21-carbonsäure γ-Lacton |
| 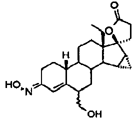 | (E/Z)-3-(Hydroxyimino)-17β-hydroxy-6α-hydroxymethyl-18-methyl-15α,16α-methylen-19-nor-17α-pregna-4-en-21-carbonsäure γ-Lacton |
| | (E/Z)-3-(Hydroxyimino)-17β-hydroxy-6β-hydroxymethyl-18-methyl-15α,16α-methylen-19-nor-17α-pregna-4-en-21-carbonsäure γ-Lacton |
| 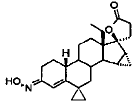 | (E/Z)-3-(Hydroxyimino)-6,6-(1,2-Ethandiyl)-17β-hydroxy-18-methyl-15α,16α-methylen-19-nor-17α-pregna-4-en-21-carbonsäure γ-Lacton |
| 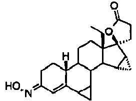 | (E/Z)-3-(Hydroxyimino)-17β-hydroxy-6,7α,15α,16α-bismethylen-19-nor-17α-pregna-4-en-21-carbonsäure γ-Lacton |
| | (E/Z)-3-(Hydroxyimino)-17β-hydroxy-6β,7β,15α,16α-bismethylen-19-nor-17α-pregna-4-en-21-carbonsäure γ-Lacton |
| 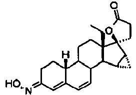 | (E/Z)-3-(Hydroxyimino)-17β-hydroxy-18-methyl-15α,16α-methylen-19-nor-17α-pregna-4,6-dien-21-carbonsäure γ-Lacton |
| 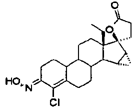 | (E/Z)-3-(Hydroxyimino)-4-Chlor-17β-hydroxy-18-methyl-15α,16α-methylen-19-nor-17α-pregna-4-en-21-carbonsäure γ-Lacton |

Aufgrund ihrer gestagenen Wirksamkeit können die neuen Verbindungen mit der allgemeinen chemischen Formel I allein oder in Kombination mit Estrogen in Arzneimitteln zur Kontrazeption verwendet werden.

Die erfindungsgemäßen Derivate eignen sich daher insbesondere zur Herstellung eines Arzneimittels zur oralen Kontrazeption und zur Behandlung von prä-, peri- und postmenopausalen Beschwerden, einschließlich der Verwendung in Präparaten für die Hormon-Substitutionstherapie (HRT).

Wegen ihres günstigen Wirkungsprofils sind die erfindungsgemäßen Derivate außerdem besonders gut geeignet zur Behandlung prämenstrueller Beschwerden, wie Kopfschmerzen, depressiver Verstimmungen, Wasserretention und Mastodynie.

Besonders bevorzugt ist die Verwendung der erfindungsgemäßen Derivate zur Herstellung eines Arzneimittels mit gestagener, bevorzugt auch antimineralcorticoider und neutraler bis leicht androgener Wirkung.

Eine Behandlung mit den erfindungsgemäßen Derivaten findet bevorzugt am Menschen statt, kann aber auch an verwandten Säugetierspezies, wie beispielsweise an Hund und Katze, durchgeführt werden.

Zur Verwendung der erfindungsgemäßen Derivate als Arzneimittel werden diese mit mindestens einem geeigneten pharmazeutisch unbedenklichen Zusatzstoff, beispielsweise Trägerstoff, kombiniert. Der Zusatzstoff ist beispielsweise für die parenterale, vorzugsweise orale, Applikation geeignet. Es handelt sich dabei um pharmazeutisch geeignete organische oder anorganische inerte Zusatzmaterialien, wie zum Beispiel, Wasser, Gelatine, Gummi arabicum, Milchzucker, Stärke, Magnesiumstearat, Talk, pflanzliche Öle, Polyalkylenglykole usw. Die Arzneimittel können in fester Form, zum Beispiel als Tabletten, Dragees, Suppositorien, Kapseln oder in flüssiger Form, zum Beispiel als Lösungen, Suspensionen oder Emulsionen, vorliegen. Gegebenenfalls enthalten sie darüber hinaus Hilfsstoffe, wie Konservierungs-, Stabilisierungs-, Netzmittel oder Emulgatoren, Salze zur Veränderung des osmotischen Druckes oder Puffer. Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden. Es ist auch möglich, die erfindungsgemäßen Derivate in ein transdermales System einzuarbeiten und sie damit transdermal zu applizieren. Für die orale Applikation kommen insbesondere Tabletten, Dragees, Kapseln, Pillen, Suspensionen oder Lösungen in Frage.

Als Applikationswege kommen weiterhin beispielsweise eine intravaginale oder intrauterine Gabe in Frage. Diese kann durch physiologisch verträgliche Lösungen, wie z.B. einer wässrigen oder öligen Lösung mit oder ohne geeigneten Löslichkeitsvermittler, Dispersionsmittel oder Emulgatoren erfolgen. Als geeignete Öle kommen beispielsweise Erdnussöl, Baumwollsamenöl, Rizinusöl oder Sesamöl in Frage. Die Auswahl ist damit keineswegs darauf beschränkt.

Für die intravaginale oder intrauterine Gabe können spezielle Systeme wie ein intravaginales System (z.B. Vaginalring, VRS) oder ein intrauterines System (IUS) verwendet werden, die eine aktive Substanz der vorliegenden Erfindung aus einem Reservoir auch über eine längere Zeit (z. B. 1, 2, 3, 4 oder 5 Jahre) freisetzen.

Als Beispiel für ein intrauterines System sei stellvertretenderweise MIRENA^{®} angeführt. Hierbei handelt es sich um ein T-förmiges, Levonorgestrel freisetzendes intrauterines Sytem der BAYER SCHERING PHARMA AG.

Weiterhin kann eine Applikation über ein implantiertes Depotsystem aus einem inerten Trägermaterial wie z.B. einem biologisch abbaubaren Polymer oder einem synthetischen Silikon-Polymer erfolgen. Diese Depotsysteme setzten den Wirkstoff kontrolliert über einen längeren Zeitraum frei (z.B. 3 Monate bis 3 Jahre) und werden subkutan implantiert.

Die Dosierung der erfindungsgemäßen Derivate in Kontrazeptionspräparaten soll 0,01 bis 10 mg pro Tag betragen. Die Tagesdosis bei der Behandlung prämenstrueller Beschwerden liegt bei etwa 0,1 bis 20 mg. Die erfindungsgemäßen gestagenen Derivate werden in Kontrazeptionspräparaten sowie in den Arzneimitteln zur Behandlung prämenstrueller Beschwerden vorzugsweise oral appliziert. Die tägliche Dosis wird vorzugsweise einmalig verabreicht. Die vorstehend genannten Dosierungen beziehen sich auf orale Verabreichungsformen.

Bei Verwendung einer Depotformulierung wird die entsprechende, zu den vorstehend genannten oralen Dosierungen equivalente Dosierung kontinuierlich pro Tag aus den längerfristig eingesetzten oben beschriebenen Depotsystemen freigesetzt.

Aus einer Depotformulierung, zum Beispiel aus einem IUS, wird täglich eine Menge von 0,005 bis 10 mg einer Verbindung der allgemeinen Formel 1 freigesetzt.

Die gestagenen und estrogenen Wirkstoffkomponenten werden in Kontrazeptionspräparaten vorzugsweise zusammen oral appliziert. Die tägliche Dosis wird vorzugsweise einmalig verabreicht.

Als Estrogene kömmen synthetische Estrogene, vorzugsweise Ethinylestradiol, aber auch Mestranol, sowie natürliche Estrogene, einschließlich Phytoestrogene, in Betracht.

Das Estrogen wird in einer täglichen Menge verabreicht, die der pharmakologischen Wirkung von 0,01 bis 0,04 mg Ethinylestradiol entspricht. Diese Menge bezieht sich auf eine orale Verabreichungsform. Wird eine anderer Verabreichungsweg gewählt ist eine entsprechende, zur vorstehend genannten oralen Dosierung equivalente Dosierungsmenge zu verwenden.

Als Estrogene in den Arzneimitteln zur Behandlung von prä-, peri- und postmenopausalen Beschwerden sowie für die Hormon-Substitutionstherapie kommen in erster Linie natürliche Estrogene zur Anwendung, vor allem das Estradiol, aber auch die Ester von Estradiol, beispielsweise Estradiolvalerat, oder auch konjugierte Estrogene (CEEs = Conjugated Equine Estrogens).

Die gestagene, antimineralcorticoide und androgene bzw. antiandrogene Wirkung der erfindungsgemaßen Verbindungen wurde mit den folgenden Methoden untersucht:

### 1. Progesteronrezeptor-Bindungstest:

Unter Verwendung von Cytosol aus Progesteronrezeptor-exprimierenden Insektenzellen (Hi5) wurde die kompetitiive Bindefähigkeit an den Progesteronrezeptor ermittelt über die Fähigkeit, ³H-Progesteron als Bezugssubstanz vom Rezeptor zu verdrängen. Verfügt eine Verbindung über eine Progesteron entsprechende Affinität, entspricht das dem Kompetitionsfaktor (KF) von 1. KF-Werte größer als 1 zeichnen sich durch eine geringere, KF-Werte kleiner als 1 durch eine höhere Affinität zum Progesteronrezeptor aus.

### 2. Mineralocorticoidrezeptor-Bindungstest:

Der Test erfolgte analog zu 1., mit folgenden Modifikationen: Zum Einsatz kam Cytosol aus Mineralocorticoidrezeptor-exprimierenden Insektenzellen (Hi5), die Bezugssubstanz war ³H-Aldosteron.

### 3. Androgenrezeptor-Bindungstest:

Der Test erfolgte analog zu 1., mit folgenden Modifikationen: Zum Einsatz kam Cytosol aus Androgenrezeptor-exprimierenden Insektenzellen (Hi5), die Bezugssubstanz war ³H-Testosteron.

Die Ergebnisse der Bindungstests sowie das Verhältnis der Kompetitionsfaktoren KF(PR) und KR(MR) sind in Tabelle 1 wiedergegeben, wobei zum Vergleich Rezeptorbindungswerte auch von Drospirenon als Bezugssubstanz A angegeben sind.

### 4. Bestimmung der gestagenen Wirkung mithilfe von Transaktivierungstests:

Zur Kultivierung der für den Assay verwendeten Zellen wurde als Kultivierungsmedium DMEM (Dulbecco's Modified Eagle Medium: 4500 mg/ml Glukose; PAA, #E15-009) mit 10% FCS (Biochrom, S0115, Charge #615B), 4 mM L-Glutamin, 1 % Penicillin/Streptomycin, 1 mg/ml G418 und 0,5 µg/ml Puromycin verwendet.

Reporter-Zelllinien (CHO K1 Zellen stabil transfiziert mit einem Fusionsprotein aus der PR-Ligandenbindungsdomäne und einer Gal4-Transaktivierungsdomäne sowie einem Reporterkonstrukt, das die Luciferase unter der Kontrolle eines Gal4-responsiven Promotors enthielt) wurden in einer Dichte von 4 x 10⁴ Zellen pro Vertiefung in weißen, undurchsichtigen Gewebekulturplatten mit jeweils 96 Vertiefungen angezüchtet (PerkinElmer, #P12-106-017) und in Kultivierungsmedium mit 3 % DCC-FCS (Aktivkohle behandeltes Serum, zur Entfernung im Serum enthaltener störender Komponenten) gehalten. Die zu untersuchenden Verbindungen wurden acht Stunden später zugegeben, und die Zellen wurden mit den Verbindungen 16 Stunden lang inkubiert. Die Versuche wurden dreifach ausgeführt. Am Ende der Inkubation wurde das Effektor enthaltende Medium entfernt und durch Lysis-Puffer ersetzt. Nachdem Luciferase-Assay-Substrat (Promega, #E1501) zugegeben worden war, wurden die Platten mit den 96 Vertiefungen dann in ein Mikroplatten-Luminometer (Pherastar, BMG labtech) eingeführt, und die Lumineszenz wurde gemessen. Die IC₅₀-Werte wurden unter Verwendung einer Software zur Berechnung von Dosis-Wirkungsbeziehungen ausgewertet. In Tabelle 1 sind Versuchsergebnisse und zum Vergleich entsprechende Ergebnisse von Drospirenon als Bezugssubstanz A wiedergegeben.

Soweit die Herstellung der Ausgangsverbindungen hier nicht beschrieben ist, sind diese dem Fachmann bekannt oder analog zu bekannten Verbindungen oder hier beschriebenen Verfahren herstellbar. Die Isomerengemische können nach üblichen Methoden, wie beispielsweise Kristallisation, Chromatographie oder Salzbildung, in die einzelnen Verbindungen aufgetrennt werden. Die Herstellung der Salze erfolgt in üblicher Weise, indem man eine Lösung der Verbindungen mit der allgemeinen chemischen Formel I mit der äquivalenten Menge oder einem Überschuss einer Base oder Säure, die sich gegebenenfalls in Lösung befindet, versetzt, gegebenenfalls den Niederschlag abtrennt oder in üblicher Weise die Lösung aufarbeitet.

Die Darstellung der Verbindungen der allgemeinen Formel I ausgehend von Verbindungen der allgemeinen Formel 1 (Schema 2) erfolgt nach den in Schema 1 angegebenen Verfahren, worin R⁴, R^{6a}, R^{6b}, R⁷, R¹⁸ und Z die vorgenannten Bedeutungen haben und
R⁶, R⁷ in 5 und 6 gemeinsam ein Sauerstoff oder eine Methylengruppe,
U ein Sauerstoffatom, zwei Alkoxygruppen OR¹⁹, eine C₂-C₁₀-Alkylen-α,ω-dioxygruppe, die geradkettig oder verzweigt sein kann, wobei R¹⁹ für einen C₁-C₂₀-Alkylrest steht,
R²⁰ einen C₁-C₂₀-Alkylrest,
X eine NR^{21a}R^{21b}-Gruppe, eine Alkoxygruppen OR²²
R^{21a}, R^{21b} gleich oder verschieden sind und Wasserstoff, C₁-C₁₀-Alkyl oder gemeinsam eine C₄-C₁₀-α,ω-Alkylengruppe, die geradkettig oder verzweigt sein kann,
R²² einen C₁-C₂₀-Alkylrest,
bedeuten.

Die Verbindungen 2 und 3 in Schema 1 tragen jeweils eine Doppelbindung zwischen C5 und C6 oder C5 und C10 sowie eine weitere Doppelbindung zwischen C2 und C3 oder C3 und C4.

Die Verbindungen 7 bis 9 in Schema 1 tragen jeweils eine Doppelbindung zwischen C4 und C5 oder C5 und C6 oder C5 und C10.

Dem Fachmann selbstverständlich ist, dass bei den Beschreibungen der synthetischen Transformationen immer vorausgesetzt wird, dass gegebenenfalls am Steroidgerüst vorhandene sonstige funktionelle Gruppen in geeigneter Form geschützt sind.

Die Einführung einer 6,7-Doppelbindung zu Verbindungen der allgemeinen Formeln **4, 13** oder **18** erfolgt über Bromierung der jeweiligen 3,5-Dienolether **3, 12** oder **17** sowie anschließende Bromwasserstoffabspaltung (siehe z. B. J. Fried, J.A. Edwards, Organic Reactions in Steroid Chemistry, von Nostrand Reinhold Company 1972, S. 265-374).

Die Dienoletherbromierung der Verbindungen **3, 12** oder **17** kann z. B. analog der Vorschrift aus Steroids 1, 233 (1963) erfolgen. Die Bromwasserstoffabspaltung zu Verbindungen der allgemeinen Formeln **4, 13** oder **18** gelingt durch Erhitzen der 6-Bromverbindung mit basischen Reagenzien, wie z. B. LiBr oder Li₂CO₃ in aprotischen Lösungsmitteln wie Dimethylformamid bei Temperaturen von 50-120°C oder aber indem die 6-Bromverbindungen in einem Lösungsmittel wie Collidin oder Lutidin erhitzt werden. Die Einführung eines Substituenten R⁴ kann zum Beispiel ausgehend von einer Verbindung der Formel **6, 11, 13, 14, 16** oder **18** durch Epoxidierung der 4,5-Doppelbindung mit Wasserstoffperoxid unter alkalischen Bedingungen und Umsetzung der entstandenen Epoxide in einem geeigneten Lösungsmittel mit Säuren der allgemeinen Formel H-R⁴ erfolgen, wobei R⁴ ein Halogenatom, vorzugsweise Chlor oder Brom, sein kann. Verbindungen, in denen R⁴ die Bedeutung von Brom besitzt lassen sich beispielsweise mit 2,2-Difluor-2-(fluorsulfonyl)essigsäuremethylester in Dimethylformamid in Gegenwart von Kupfer(I)iodid zu Verbindungen umsetzen, in denen R⁴ die Bedeutung Fluor besitzt. Alternativ kann ausgehend von einer Verbindung der Formel **6, 11, 13, 14, 16** oder **18** durch Umsetzung mit Sulfurylchlorid oder Sulfurylbromid in Gegenwart einer geeigneten Base wie beispielsweise Pyridin, direkt Halogen mit R⁴ in der Bedeutung Chlor oder Brom eingeführt werden.

Verbindung **4** wird durch Methenylierung der 6,7-Doppelbindung nach bekannten Verfahren z.B. mit Dimethylsulfoxoniummethylid (siehe z. B. DE-A 11 83 500, DE-A 29 22 500, EP-A 0 019 690, US-A 4,291, 029; J.Am.Chem.Soc. 84, 867 (1962)) in eine Verbindung **5** (R⁶, R⁷ gemeinsam eine Methylengruppe) umgewandelt, wobei ein Gemisch der α- und β-Isomeren erhalten wird, das z. B. durch Chromatographie in die einzelnen Isomeren getrennt werden kann.

Verbindungen vom Typ **5** können wie in den Beispielen beschrieben oder analog zu diesen Vorschriften unter Verwendung analoger zu den dort beschriebenen Reagenzien erhalten werden.

Die Synthese der spirocyclischen Verbindung **18** (R^{6a}, R^{6b} bilden gemeinsam 1,2-Ethandiyl) geht von Verbindungen **11** oder **14** aus, welche zunächst in ein 3-Amino-3,5-dien-Derivat **15** (X= NR^{21a}R^{21b}) überführt werden. Durch Umsetzung mit Formalin in alkoholischer Lösung wird das 6-Hydroxymethylen-Derivat **16** (R⁶ = Hydroxymethylen) erhalten. Nach Überführung der Hydroxygruppe in eine Fluchtgruppe wie etwa ein Mesylat, Tosylat oder auch Benzoat lässt sich Verbindung **18** durch Umsetzung mit Trimethylsulfoxoniumiodid unter Verwendung von Basen wie etwa Alkalihydroxyden, Alkalialkoholaten in geeigneten Lösemitteln wie etwa Dimethylsulfoxyd darstellen.

Zur Einführung einer 6-Methylengruppe kann Verbindung **16** (R⁶ = Hydroxymethylen) mit z.B. Salzsäure in Dioxan/Wasser dehydratisiert werden. Auch nach Überführung der Hydroxygruppe in eine Fluchtgruppe wie etwa ein Mesylat, Tosylat oder auch Benzoat lässt sich Verbindung **18** (R^{6a}, R^{6b} gemeinsam Methylen) erzeugen (siehe DE-A 34 02 3291, EP-A. 0 150 157, US-A 4,584,288; J. Med. Chem. 34, 2464 (1991)).

Eine weitere Möglichkeit zur Herstellung von 6-Methylenverbindungen **18** besteht in der direkten Umsetzung der 4(5) ungesättigten 3-Ketone wie Verbindung **16** (R⁶ = Wasserstoff) mit Acetalen des Formaldehyds in Gegenwart von Natriumacetat mit z. B. Phosphoroxychlorid oder Phosphorpentachlorid in geeigneten Lösungsmitteln wie Chloroform (siehe z. B. K. Annen, H. Hofmeister, H. Laurent und R. Wiechert, Synthesis 34 (1982)).

Die 6-Methylenverbindungen können zur Darstellung von Verbindungen der allgemeinen Formel **18,** in denen R^{6a} gleich Methyl ist und R^{6b} und R⁷ gemeinsam eine zusätzliche Bindung bilden, genutzt werden.

Hierzu kann man z. B. ein in Tetrahedron 21, 1619 (1965) beschriebenes Verfahren anwenden, bei dem eine Isomerisierung der Doppelbindung durch Erwärmen der 6-Methylenverbindungen in Ethanol mit 5% Palladium-Kohle-Katalysator, der entweder mit Wasserstoff oder durch Erwärmen mit einer geringen Menge Cyclohexen vorbehandelt wurde, erzielt werden. Die Isomerisierung kann auch mit einem nicht vorbehandelten Katalysator erfolgen, wenn zur Reaktionsmischung eine geringe Menge Cyclohexen zugesetzt wird. Das Auftreten geringer Anteile hydrierter Produkte kann durch Zugabe eines Überschusses an Natriumacetat verhindert werden.

Alternativ kann die Verbindung **17** (X= OR²²) als Vorstufe verwendet werden. Die direkte Darstellung von 6-Methyl-4,6-dien-3-on-Derivaten ist beschrieben (siehe K. Annen, H. Hofmeister, H. Laurent und R. Wiechert, Lieb. Ann. 712 (1983)).

Verbindungen **18,** in denen R^{6b} eine α-Methylfunktion darstellt, können aus den 6-Methylenverbindungen **(18:** R^{6a}, R^{6b} gemeinsam Methylen) durch Hydrierung unter geeigneten Bedingungen dargestellt werden. Die besten Ergebnisse (selektive Hydrierung der exo-Methylenfunktion) werden durch Transfer-Hydrierung erreicht (J. Chem.Soc. 3578 (1954)). Erhitzt man die 6-Methylenderivate **18** in einem geeigneten Lösungsmittel, wie z. B. Ethanol, in Gegenwart eines Hydriddonators, wie z. B. Cyclohexen, so kommt man in sehr guten Ausbeuten zu 6α-Methylderivaten. Geringe Anteile an 6β- Methylverbindung können sauer isomerisiert werden (Tetrahedron 1619 (1965)).

Auch die gezielte Darstellung von 6β-Methylverbindungen ist möglich. Hierfür werden die 4-En-3-one wie etwa Verbindung **16 z.** B. mit Ethylenglycol, Trimethylorthoformiat in Dichlormethan in Gegenwart katalytischer Mengen einer Säure, z. B. p-Toluolsulfonsäure, zu den entsprechenden 3-Ketalen umgesetzt. Während dieser Ketalisierung isomerisiert die Doppelbindung in die Position 5. Eine selektive Epoxidierung dieser 5-Doppelbindung gelingt z. B. durch Verwendung organischer Persäuren, z. B. m-Chlorperbenzoesäure, in geeigneten Lösungsmittel wie Dichlormethan. Alternativ hierzu kann die Epoxidierung auch mit Wasserstoffperoxid in Gegenwart von z. B. Hexachloraceton oder 3- Nitrotrifluoracetophenon erfolgen. Die gebildeten 5,6α-Epoxide können dann unter Verwendung entsprechender Alkylmagnesiumhalogenide oder Alkyllithiumverbindungen axial geöffnet werden. Man gelangt so zu 5α-Hydroxy-6β-Alkylverbindungen; Die Spaltung der 3-Ketoschutzgruppe kann unter Erhalt der 5α-Hydroxyfunktion durch Behandeln unter milden sauren Bedingungen (Essigsäure oder 4 n Salzsäure bei 0°C) erfolgen. Basische Eliminierung der 5α-Hydroxyfunktion mit z. B. verd. wäßriger Natronlauge ergibt die 3-Keto-4-en-Verbindungen mit einer β-ständigen 6-Alkylgruppe. Alternativ hierzu ergibt die Ketalspaltung unter drastischeren Bedingungen (wäßrige Salzsäure oder eine andere starke Säure) die entsprechenden 6α-Alkylverbindungen.

Die Einführung einer 7-Alkyl, 7-Alkenyl- oder 7-Alkinylgruppe zu Verbindungen der allgemeinen Formel **14** erfolgt durch 1,6-Addition einer entsprechenden Metallorganischen Verbindung an die Vorstufe der allgemeinen Formel **13** unter der Einwirkung von Kupfersalzen. Bevorzugt sind zweiwertige Metalle wie Magnesium und Zink, als Gegenion sind bevorzugt Chlor, Brom und Iod. Als Kupfersalze eignen sich ein- oder zweiwertige Kupferverbindungen wie beispielsweise Kupferchlorid, Kupferbromid oder Kupferacetat. Die Reaktion erfolgt in einem inerten Lösungsmittel wie beispielsweise Tetrahydrofuran, Diethylether oder Dichlormethan.

Die erhaltenen Verbindungen **6, 11, 13, 14, 16, 18** oder **20** in denen Z für ein Sauerstoffatom steht, können durch Umsetzung mit Hydroxylaminhydrochlorid Alkyloxyaminhydrochloriden oder Sulfonylhydrazinen in Gegenwart eines tertiären Amins bei Temperaturen zwischen -20 und +40°C in ihre entprechenden E/Z-konfigurierten Oxime oder Sulfonylhydrazone überführt werden (allgemeine Formel **I** mit Z in der Bedeutung von =NOR¹, =NNHSO₂R¹)). Geeignete tertiäre Basen sind beispielsweise Trimethylamin, Triäthylamin, Pyridin, N,N-Dimethylaminopyridin, 1,5- Diazabicyclo[4.3.0]non-5-en (DBN) und 1,5- Diazabicyclo[5.4.0]undec-5-en (DBU), wobei Pyridin bevorzugt ist. Ein analoges Verfahren ist beispielsweise in WO 98/24801 für die Herstellung entsprechender 3-Oxyimino-Derivate des Drospirenons beschrieben.

Die Entfernung der 3-Oxogruppe zur Herstellung eines Endprodukts der allgemeinen Formel I mit Z in der Bedeutung von zwei Wasserstoffatomen kann beispielsweise nach der in DE-A 28 05 490 angegebenen Vorschrift durch reduktive Spaltung eines Thioketals der 3-Ketoverbindung erfolgen auf einer geeigneten Vorstufe wie beispielsweise Verbindungen der allgemeinen Formeln **6, 11, 13, 14, 16, 18** oder **20** erfolgen.

Die Spirolactonbildung zu Verbindungen der allgemeinen Formeln **6** oder **11** erfolgt ausgehend von den entsprechenden 17-Hydroxypropenylverbindungen **5** oder **10** durch Oxidation. Als Oxidationsverfahren seien beispielsweise genannt die Oxidation nach Jones, die Oxidation mit Kaliumpermanganat beispielsweise in einem wässrigen System aus tert.-Butanol und Natriumdihydrogenphosphat, die Oxidation mit Natriumchlorit in wässrigem tert.-Butanol gegebenenfalls in Gegenwart eines Chlorfängers wie z.B. 2-Methyl-2-buten oder durch Oxidation mit Braunstein.

Alternativ läßt sich das Spirolacton direkt aus den Ketonen der allgemeinen Formeln **1** oder **7** gegebenenfalls auch nach Spaltung der Enolether in **1** bzw. Ketale in **7** nach dem von Georges Sturtz et. al. in Tetrahedron Letters 47 (1976) beschriebenen Verfahren direkt einführen. 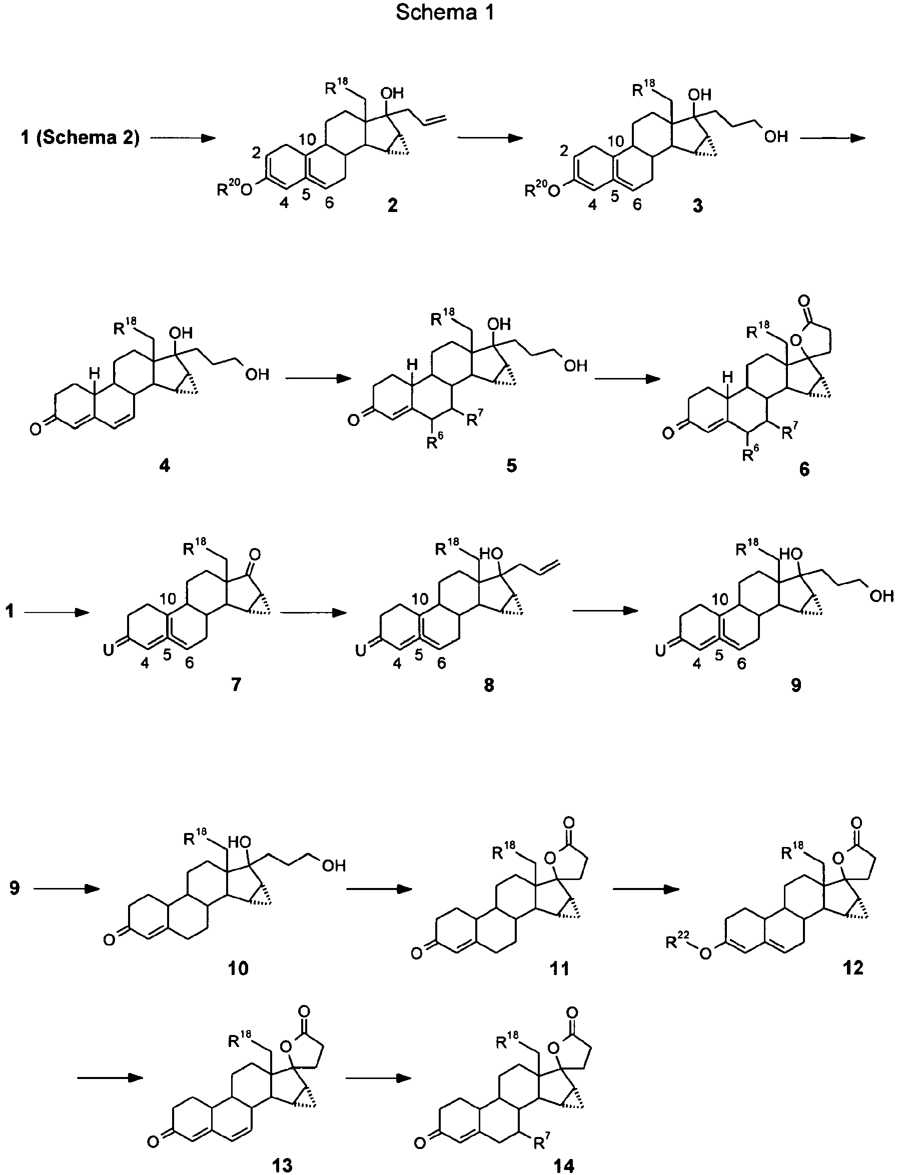 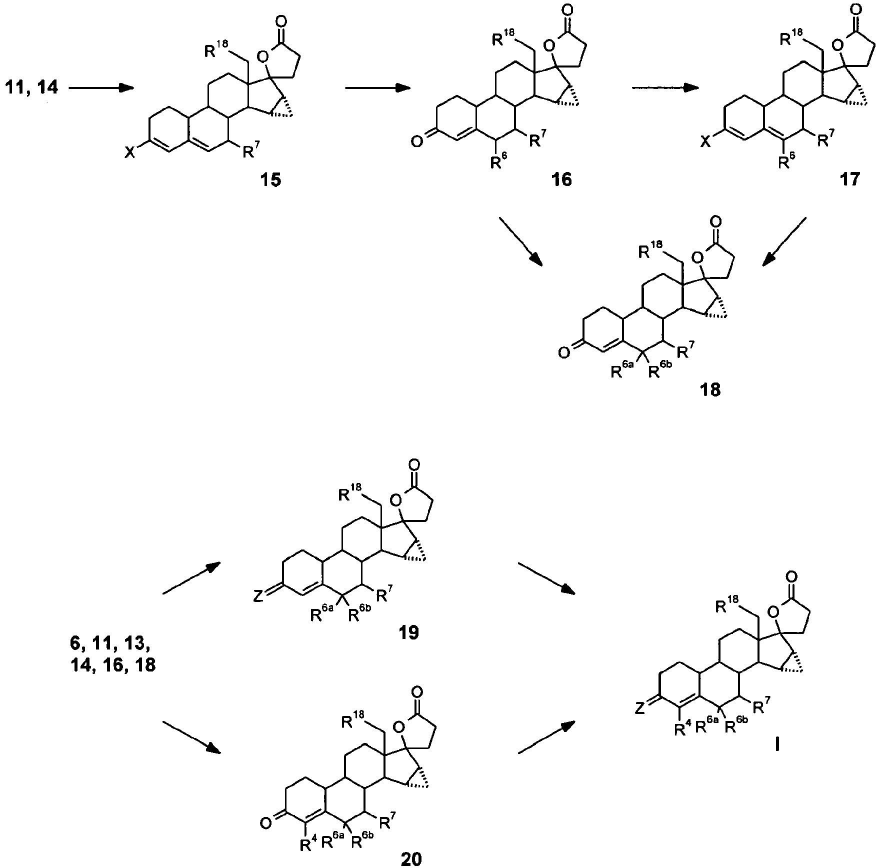 Die Verbindung 1 in Schema 2 trägt jeweils eine Doppelbindung zwischen C5 und C6 oder C5 und C10 sowie eine weitere Doppelbindung zwischen C2 und C3 oder C3 und C4.

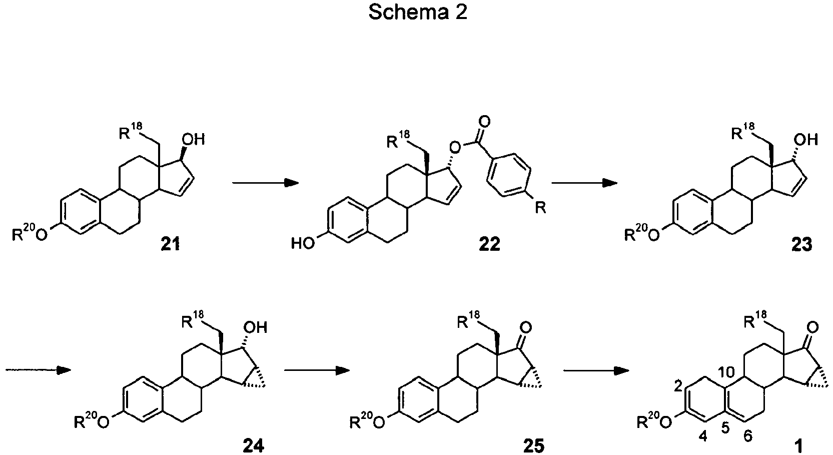

Die nachfolgenden Beispiele dienen der näheren Erläuterung der Erfindung ohne diese auf die angeführten Beispiele einzugrenzen:

### Beispiel 1: (17-Spirolactonisierung mit Braunstein)

### 17β-Hydroxy-15α,16α-methylen-19-nor-17α-pregna-4-en-3-on-21-carbonsäure γ-Lacton

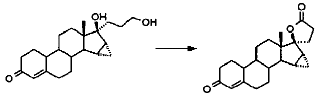

Die Lösung von 150 mg der nach Beispiel 1 a dargestellten Verbindung in 7 ml Dichlormethan versetzt man mit 1,14 g Braunstein und rührt ca. 16 Stunden bei 23°C. Man filtriert über Celite und isoliert nach Einengen und Chromatographie 125 mg der Titelverbindung.

¹H-NMR (CDCl₃): δ= 0,52 (1H), 0,57 (1H), 0,75 (1H), 1,03 (1H), 1,15 (1H), 1,22 (3H), 1,23-134 (3H), 1,43-1,65 (3H), 1,76-1,86 (2H), 1,97-2,56 (11H), 5,84 (1H) ppm.

### Beispiel 1 a: (3-Ketalspaltung)

### 17α(Z)-(3'-Hydroxyprop-1'-yl)-15α,16α-methylen-17β-hydroxyestra-4-en-3-on

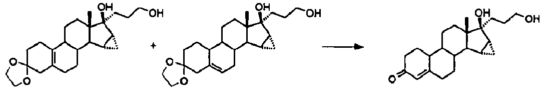

Die Lösung von 880 mg der nach Beispiel 1 b dargestellten Verbindungen in 35 ml Aceton versetzt man mit 1,8 µl einer 4N Salzsäure und rührt 1 Stunde bei 23°C. Man gießt in gesättigte Natriumhydrogencarbonatlösung, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie. Isoliert werden 580 mg der Titelverbindung.

### Beispiel 1b: (Hydroborierung)

### 17α(Z)-(3'-Hydroxyprop-1'-yl)-15α,16α-methylen-17β-hydroxyestra-5-en-3-on-3-ethylenketal und 17α(Z)-(3'-Hydroxyprop-1'-yl)-15α,16α-methylen-17β-hydroxyestra-5(10)-en-3-on-3-ethylenketal

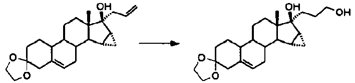

Die Lösung von 643 mg der nach Beispiel 1c dargestellten Verbindung in 7,5 ml Tetrahydrofuran versetzt man mit 10,5 ml einer 0,5 molaren Lösung von 9-Borabicyclononan in Tetrahydrofuran und rührt 4 Stunden bei 23°C. Man kühlt auf 4°C, versetzt mit 4,6 ml einer 5%-igen Natriumhydroxidlösung, 1,2 ml einer 30%-igen Wasserstoffperoxidlösung und rührt 15 Stunden bei 23°C nach. Man extrahiert mit Ethylacetat, wäscht die vereinigten organischen Extrakte mit Wasser, gesättigter Natriumthiosulfatlösung, gesättigter Natriumchloridlösung und trocknet über Natriumsulfat. Nach Filtration und Lösungsmittelabzug isoliert man 880 mg der Titelverbindung, die ohne Reinigung weiter umgesetzt wird.

### Beispiel 1c: (17-Allyladdition)

### 17α-(2'-propen-1'-yl)-15α,16α-methylen-17β-hydroxyestra-5-en-3-on-3-ethylenketal und 17α-(2'-propen-1'-yl)-15α,16α-methylen-17β-hydroxyestra-5(10)-en-3-on-3-ethylenketal

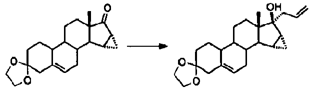

Die Lösung von 600 mg der nach Beispiel 1d dargestellten Verbindung in 10 ml Dichlormethan versetzt man bei 4°C mit 4,38 ml einer 1 molaren Lösung von Allylmagnesiumbromid in Diethylether, rührt 1 Minute und gießt in gesättigte Ammoniumchloridlösung. Man extrahiert mit Ethylacetat, wäscht die vereinigten organischen Extrakte mit gesättigter Natriumchloridlösung und trocknet über Natriumsulfat. Nach Filtration und Lösungsmittelabzug isoliert man 690 mg der Titelverbindung, die ohne Reinigung weiter umgesetzt wird.

### Beispiel 1d: (Oxidation von 17-OH)

### 15α,16α-Methylen-estra-5-en-3,17-dion-3-ethylenketal und 15α,16α-methylen-estra-5(10)-en-3,17-dion-3-ethylenketal

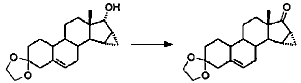

Die Lösung von 1,06 g der nach Beispiel 1e dargestellten Verbindungen in 32 ml Dichlormethan versetzt man einer Spatelspitze Molekularsieb 4A, 700 mg N-Methylmorpholino-N-oxid, 90 mg Tetrabutylammoniumperruthenat und rührt bei 23°C ca. 16 Stunden. Man ent ein und reinigt den Rückstand durch Chromatographie. Isoliert werden 878 mg der Titelverbindungen.

### Beispiel 1e: (3-Enolether zu Ethylenketal)

### 15α,16α-Methylen-17α-hydroxyestra-5-en-3-on-3-ethylenketal und 15α,16α-Methylen-17α-hydroxyestra-5(10)-en-3-on-3-ethylenketal

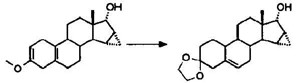

Die Lösung von 500 mg der nach Beispiel 1f dargestellten Verbindung in 10 ml Tetrahydrofuran versetzt man mit 10 ml Ethylenglycol, 4,4 mg p-Toluolsulfonsäure Hydrat und rührt bei 23°C 2 Stunden. Man gießt in gesättigte Natriumhydrogencarbonatlösung, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie. Isoliert werden 359 mg der Titelverbindung.

### Beispiel 1f: (Birch) 3-Methoxy-15α,16α-methylen-17α-hydroxyestra-2,5(10)-dien

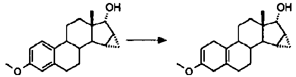

597 ml Ammoniak werden bei -75°C mit 9,91 g Lithium versetzt und innerhalb von 1 Stunde die Lösung von 24,6 g der nach Beispiel 1g dargestellten Verbindung in 1,2 l Tetrahydrofuran zugetropft. Man versetzt mit 720 ml Ethanol läßt nach 1 stunde auf -50°C erwärmen und rührt weitere 2 Stunden. Anschließend versetzt man mit 600 ml Wasser, läßt auf 23°C erwärmen, extrahiert mehrfach mit Ethylacetat, wäscht die vereinigten organischen Extrakte mit gesättigter Natriumchloridlösung und trocknet über Natriumsulfat. Nach Filtration und Lösungsmittelabzug isoliert man 27,1 g der Titelverbindung, die ohne Reinigung weiter umgesetzt wird.

### Beispiel 1g: (Simmons Smith)

### 3-Methoxy-15α,16α-methylen-17α-hydroxyestra-1,3,5(10)-trien

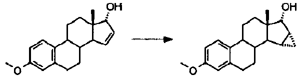

Eine Suspension aus 1,5 g Kupfer(II)acetat in 900 ml Diethylether versetzt man mit 86,6 g Zinkstaub und erhitzt 10 Minuten unter Rückfluß. Anschließend versetzt man mit 11,7 ml Diiodmethan und erhitzt weitere 30 Minuten unter Rückfluß. Man gibt die Lösung von 37,6 g der nach Beispiel 1h dargestellten Verbindung in 100 ml Tetrahydrofuran sowie über insgesamt 40 Stunden verteilt insgesamt weitere 35 ml Diiodmethan zu. Das erkaltete Gemisch filtriert man über Celite, wäscht das Filtrat mit gesättigter Natriumchloridlösung und trocknet über Natriumsulfat. Den nach Filtration und Lösungsmittelabzug erhaltenen Rückstand reinigt man durch Umkristallisation. Isoliert werden 24,6 g der Titelverbindung.

### Beispiel 1 h: (Benzoatverseifung)

### 3-Methoxy-17α-hydroxyestra-1,3,5(10),15-tetraen

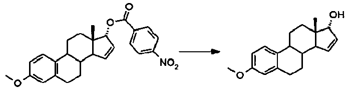

Die Lösung von 96,3 g der nach Beispiel 1i dargestellten Verbindung in 1,1 I Methanol versetzt man mit 75,5 g Kaliumcarbonat und rührt bei 50°C 2 Stunden. Man engt ein, versetzt mit Wasser, extrahiert mehrfach mit Ethylacetat, wäscht die vereinigten organischen Extrakte mit Wasser und trocknet über Natriumsulfat. Den nach Filtration und Lösungsmittelabzug erhaltenen Rückstand reinigt man durch Umkristallisation. Isoliert werden 46 g der Titelverbindung.

### Beispiel 1i: (Mitsunobu)

### 4-Nitro-benzoesäure 3-Methoxy-estra-1,3,5(10),15-tetraen-17-yl ester

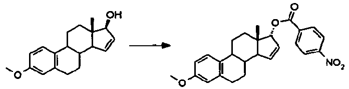

Die Lösung von 43,9 g 3-Methoxy-17β-hydroxyestra-1,3,5(10),15-tetraen in 1,6 I Tetrahydrofuran versetzt man mit 121 g Triphenylphosphin, 27,1 g 4-Nitrobenzoesäure, 30,9 ml Azodicarbonsäurediisopropylester und rührt bei 23°C 2 Stunden. Man versetzt mit gesättigter Natriumchloridlösung, extrahiert mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand nimmt man in 1,2 I Aceton auf, versetzt unter Kühlung mit 80 ml einer 30%igen Wasserstoffperoxidlösung und gießt nach 20 Minuten unter Kühlung in 600 ml einer halbkonzentrierten Natriumthiosulfatlösung ein. Man extrahiert mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Umkristallisation. Isoliert werden 52,5 g der Titelverbindung.

### Beispiel 2: (Dienonbildung aus Dienolether)

### 17β-Hydroxy-15α,16α-methylen-19-nor-17α-pregna-4,6-dien-3-on-21-carbonsäure γ-Lacton

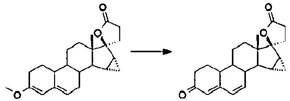

Die Lösung von 1,14 g der nach Beispiel 2a dargestellten Verbindung in 2,1 ml N-Methylpyrrolidon versetzt man bei -10°C mit 119 mg Natriumacetat, 1,2 ml Wasser und portionsweise mit insgesamt 460 g Dibromhydantoin. Nach 30 Minuten versetzt man mit 447 mg Lithiumbromid, 392 mg Lithiumcarbonat und erhitzt 2,5 Stunden bei einer Badtemperatur von 100°C. Man gießt auf ein Gemisch aus Eis und Natriumchloridlösung und saugt das ausgefallene Produkt ab. Isoliert werden 910 mg der Titelverbindung als kristallines Rohprodukt das direkt weiter umgesetzt werden kann.

¹H-NMR (CDCl₃): δ= 0,59 (1H), 0,72 (1H), 1,00 (1H), 1,10 (1H), 1,19-1,59 (5H), 1,24 (3H), 1,79 (1 H), 1,87 (1 H), 1,99-2,59 (9H), 5,80 (1 H), 6,24 (1 H), 6,38 (1 H) ppm.

### Beispiel 2a: (Dienoletherbildung)

### 17β-Hydroxy-3-methoxy-15α,16α-methylen-19-nor-17α-pregna-3,5-dien-21-carbonsäure γ-Lacton

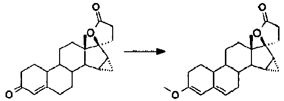

Die Lösung von 2 g der nach Beispiel 1 dargestellten Verbindung in 29 ml 2,2-Dimethoxypropan versetzt man mit 221 mg Pyridinium-p-toluolsulfonat und erhitzt 4 Stunden unter Rückfluß. Man gießt in gesättigte Natriumhydrogencarbonatlösung, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Kristallisation. Isoliert werden 1,15 g der Titelverbindung.

### Beispiel 3: (1,6-Addition (Methyl))

### 17β-Hydroxy-7α-methyl-15α,16α-methylen-19-nor-17α-pregna-4-en-3-on-21-carbonsäure γ-Lacton (A) und 17β-Hydroxy-7β-methyl-15α,16α-methylen-19-nor-17α-pregna-4-en-3-on-21-carbonsäure γ-Lacton (B)

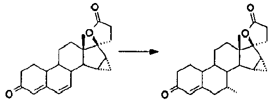

Zu der auf -30°C gekühlten Suspension von 7 mg Kupfer-(I)-chlorid in 1,2 ml Tetrahydrofuran tropft man 250 µl einer 3 molaren Lösung von Methylmagnesiumchlorid in Tetrahydrofuran und rührt noch 10 Minuten. Man kühlt auf -25°C und tropft die Lösung zu 100 mg der nach Beispiel 2 dargestellten Verbindung in 5 ml Tetrahydrofuran zu. Nach 2 Minuten gießt man auf 1 N Salzsäure, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie. Isoliert werden 23 mg der Titelverbindung A neben einem noch verunreinigten Gemisch, das Anteile der Titelverbindung B enthält.

¹H-NMR (CDCl₃) von A: δ= 0,51 (1H), 0,66 (1H), 0,81 (3H), 0,94-1,10 (2H), 1,22 (3H), 1,18-1,60 (6H), 1,74-1,91 (3H), 2,00-2,58 (10H), 5,85 (1H) ppm.

### Beispiel 4:

### 17β-Hydroxy-7α-ethyl-15α,16α-methylen-19-nor-17α-pregna-4-en-3-on-21-carbonsäure γ-Lacton (A) und 17β-Hydroxy-7β-ethyl-15α,16α-methylen-19-nor-17α-pregna-4-en-3-on-21-carbonsäure γ-Lacton (B)

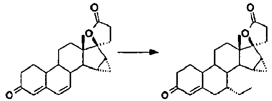

In Analogie zu Beispiel 3 setzt man 200 mg der nach Beispiel 2 dargestellten Verbindung unter Verwendung von Ethylmagnesiumchlorid um und isoliert nach Aufarbeitung und Reinigung 81 mg der Titelverbindung A neben einem noch verunreinigten Gemisch, das Anteile der Titelverbindung B enthält.

¹H-NMR (CDCl₃) von A: δ= 0,51 (1H), 0,75 (1H), 0,90 (3H), 0,95-1,10 (3H), 1,18-1,38 (4H), 1,22 (3H), 1,44 (1H), 1,50 (1H), 1,77-1,96 (4H), 2,01-2,10 (2H), 2,15 (1H), 2,22-2,54 (6H), 2,60 (1H), 5,86 (1 H) ppm.

### Beispiel 5:

### 17β-Hydroxy-7α-vinyl-15α,16α-methylen-19-nor-17α-pregna-4-en-3-on-21-carbonsäure γ-Lacton (A) und 17β-Hydroxy-7β-vinyl-15α,16α-methylen-19-nor-17α-pregna-4-en-3-on-21-carbonsäure γ-Lacton (B)

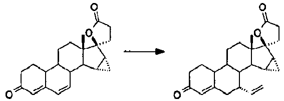

In Analogie zu Beispiel 3 setzt man 210 mg der nach Beispiel 2 dargestellten Verbindung unter Verwendung von Vinylmagnesiumchlorid um und isoliert nach Aufarbeitung und Reinigung 16 mg der Titelverbindung A neben einem noch verunreinigten Gemisch, das Anteile der Titelverbindung B enthält.

¹H-NMR (CDCl₃) von A: δ= 0,45 (1H), 0,63 (1H), 0,97-1,10 (2H), 1,16-1,36 (4H), 1,23 (3H), 1,40-1,57 (2H), 1,78-2,17 (5H), 2,22-2,54 (6H), 2,60 (1H), 2,79 (1H), 5,10 (1H), 5,18 (1 H), 5,69 (1H), 5,88 (1 H) ppm.

### Beispiel 6:

### 17β-Hydroxy-7α-cyclopropyl-15α,16α-methylen-19-nor-17α-pregna-4-en-3-on-21-carbonsäure γ-Lacton (A) und 17β-Hydroxy-7β-cyclopropyl-15α,16α-methylen-19-nor-17α-pregna-4-en-3-on-21-carbonsäure γ-Lacton (B)

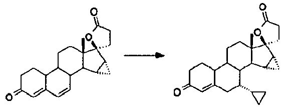

In Analogie zu Beispiel 3 setzt man 200 mg der nach Beispiel 2 dargestellten Verbindung unter Verwendung von Cyclopropylmagnesiumbromid um und isoliert nach Aufarbeitung und Reinigung 71 mg der Titelverbindung A neben einem noch verunreinigten Gemisch, das Anteile der Titelverbindung B enthält.

¹H-NMR (CDCl₃) von A: δ= -0,05 (1 H), 0,41-0,53 (4H), 0,56 (1 H), 0,99 (1 H), 1,12 (1 H), 1,16-1,32 (5H), 1,23 (3H), 1,43-1,57 (2H), 1,81-1,93 (3H), 2,02-2,21 (3H), 2,24-2,34 (2H), 2,38-2,57 (5H), 5,90 (1 H) ppm.

### Beispiel 7: (6-Hydroxymethyl)

### 17β-Hydroxy-6β-hydroxymethyl-15α,16α-methylen-19-nor-17α-pregna-4-en-3-on-21-carbonsäure γ-Lacton

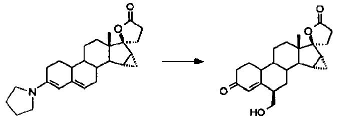

Die Lösung von 400 mg der nach Beispiel 7a dargestellten Verbindung in einem Gemisch aus 4 ml Toluol und 8 ml Ethanol versetzt man mit 400 µl einer 37%igen wässrigen Formaldehydlösung und rührt 3 Stunden bei 23°C. Man engt ein und reinigt den Rückstand durch Chromatographie. Isoliert werden 180 mg der Titelverbindung.

¹H-NMR (CDCl₃): δ= 0,52 (2H), 0,75 (1 H), 1,03 (1 H), 1,21 (3H), 1,17-1,86 (10H), 1,97-2,56 (9H), 2,68 (1 H), 3,73 (2H), 5,93 (1 H) ppm.

### Beispiel 7a: (Dienamin für 6-Alkylierung)

### 17β-Hydroxy-3-pyrrolidinyl-15α,16α-methylen-19-nor-17α-pregna-3,5-dien-21-carbonsäure γ-Lacton

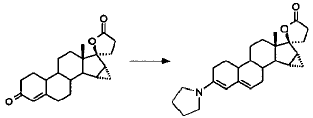

Die Lösung von 500 mg der nach Beispiel 1 dargestellten Verbindung in 5,3 ml Methanol versetzt man mit 280 µl Pyrrolidin und erhitzt 2 Stunden unter Rückfluß. Man kühlt ab, saugt den Niederschlag ab, wäscht mit wenig kaltem Methanol nach und erhält 406 mg der Titelverbindung, die man ohne zusätzliche Reinigung weiter umsetzt.

### Beispiel 8: (6-Spirocyclopropanierung (Corey))

### 6,6-(1,2-Ethandiyl)-17β-hydroxy-15α,16α-methylen-19-nor-17α-pregna-4-en-3-on-21-carbonsäure γ-Lacton

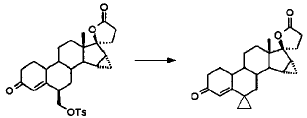

Man löst 100 mg Trimethylsulfoxoniumiodid in 1,0 ml Dimethylsulfoxid, versetzt mit 18,5 mg einer 60%igen Natriumhydrid-Dispersion und rührt 2 Stunden bei 23°C. Anschließend tropft man die Lösung von 58 mg der nach Beispiel 8a dargestellten Verbindung in 2,5 ml Dimethylsulfoxid zu und rührt weitere 3,5 Stunden bei 23°C. Man gießt in Wasser, extrahiert mehrfach mit Ethylacetat, wäscht die vereinigten organischen Extrakte mit Wasser und gesättigter Natriumchloridlösung und trocknet über Natriumsulfat. Den nach Filtration und Lösungsmittelabzug erhaltenen Rückstand reinigt man durch Chromatographie. Isoliert werden 20 mg der Titelverbindung.

¹H-NMR (CDCl₃): δ= 0,43 (1 H), 0,50 (1 H), 0,53-0,62 (2H), 0,74 (1 H), 0,85 (1 H), 1,00 (1H), 1,17-1,37 (5H), 1,24 (3H), 1,42-1,53 (2H), 1,66 (1H), 1,78-1,88 (3H), 2,02 (1H), 2,09-2,32 (4H), 2,37-2,54 (3H), 5,70 (1 H) ppm.

### Beispiel 8a: (6-Tosyloxymethyl)

### 17β-Hydroxy-6β-(p-tolylsulfonyloxymethyl)-15α,16α-methylen-19-nor-17α-pregna-4-en-3-on-21-carbonsäure γ-Lacton

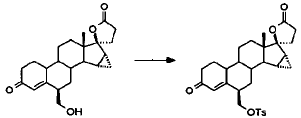

Die Lösung von 150 mg der nach Beispiel 7 dargestellten Verbindung in 7,5 ml Dichlormethan versetzt man mit 665 µl Triethylamin, 190 mg p-Toluolsulfonsäurechlorid und rührt 37 Stunden bei 23°C. Man gießt in gesättigte Natriumcarbonatlösung, extrahiert mehrfach mit Ethylacetat, wäscht die vereinigten organischen Extrakte mit Wasser und gesättigter Natriumchloridlösung und trocknet über Natriumsulfat. Den nach Filtration und Lösungsmittelabzug erhaltenen Rückstand reinigt man durch Chromatographie. Isoliert werden 131 mg der Titelverbindung.

### Beispiel 9:

### 17β-Hydroxy-6β,7β-15α,16α-bismethylen-19-nor-17α-pregna-4-en-3-on-21-carbonsäure γ-Lacton (A) und 17β-Hydroxy-6α,7α-15α,16α-bismethylen-19-nor-17α-pregna-4-en-3-on-21-carbonsäure γ-Lacton (B)

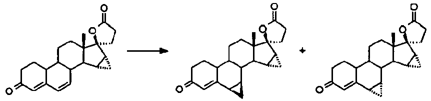

In Analogie zu Beispiel 8 setzt man 1,13 g der nach Beispiel 2 dargestellten Verbindung um und isoliert nach Aufarbeitung und Reinigung 46 mg der Titelverbindung A sowie 222 mg der Titelverbindung B.

¹H-NMR (CDCl₃) von A: δ= 0,58-0,68 (2H), 0,80-0,97 (2H), 1,09-1,37 (4H), 1,24 (3H), 1,43-1,96 (8H), 2,05-2,60 (8H), 6,15 (1H) ppm.

¹H-NMR (CDCl₃) von B: δ= 0,60 (1H), 0,64-0,74 (2H), 0,95 (1H), 1,08 (1H), 1,14-1,51 (5H), 1,25 (3H), 1,66 (1 H), 1,73-1,90 (3H), 1,98-2,58 (10H), 6,04 (1 H) ppm.
Tabelle 1

### Beispiel 10

Inerte, intrauterin implantierbare Depotsysteme aus einem biologisch abbaubaren Polymer bzw. einem synthetischen Silikon-Polymer, bestehend aus einem wirkstoffhaltigen Kern in entsprechendem Polymer-Wirkstoff-Mischungsverhältnis, umgeben von einer die gewünschte tägliche Freisetzungsrate gewährleistenden Polymermembran, werden in das Uteruslumen von Ratten verbracht. Die weiblichen Tiere werden vorher kastriert und mit Estradiol über drei Tage vorbehandelt. Die Implantate von unterschiedlicher Länge (5-20 mm) und einem begrenzten Durchmesser (1.1 bis 2 mm) verbleiben zwischen 4 und 14 Tage im Rattenuterus, um die lokale wie sy-stemische gestagene Wirkung des freigesetzten Wirkstoffes anhand verschiedener Parameter in unterschiedlichen Gewebe zu untersuchen. Folgende Parameter werden ermittelt: 1) gestagene lokale Wirkung am Uterus anhand des Uterusgewichts, der histologisch erfassbaren Epithelhöhe und der Expression gestagenregulierter Markergene (z.B. IGFBP-1); 2) gestagene systemische Wirkung an der Mamma anhand der Expression gestagenregulierter Markergene (z.B. RankL), 3) gestagene systemische Wirkung an der Hypophyse anhand des LH-Spiegels (Absenkung des estrogen-induziert erhöhten LH-Spiegels).

Die Verbindungen der vorliegenden Erfindung zeigen einen signifikanten gestagenen Effekt im Uterus der vergleichbar mit einer entsprechenden Behandlung mit einem Levonorgestrel enthaltenden Depotsystems wie MIRENA^{®} ist.

| | | Rezeptorbindung | | | | | In vitro Transaktivität | | |
|---|---|---|---|---|---|---|---|---|---|
| | | Progesteronrezeptor (PR) | | Mineralocorticoidrezeptor (MR) | Androgenrezeptor | | Progesteronrezeptor (PR) | | |
| Bsp. | Struktur | IC50 [nM] | Competition factor | Competition factor | IC50 [nM] | Competition factor | Agonis-ten EC50 [nM] | Agonisten Wirksamkeit [%] | KF PR / KF MR |
| A | 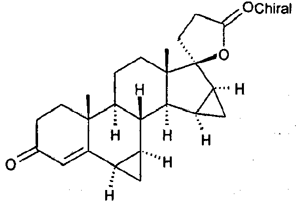 | 43,3 | 2,7 | 0.5 | 630 | 37 | 88 | 72,2 | 5,40 |
| 1 | 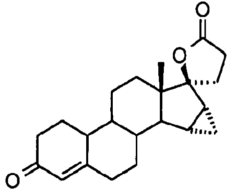 | 15 | 0,74 | 1,8 | 6 | 110,0 | 0,1 | 55,9 | 0,41 |
| 3 | 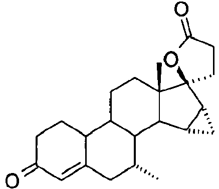 | 12,1 | 0,60 | 1,3 | 61 | 2,8 | 0,6 | 87,0 | 0,46 |
| 4 | 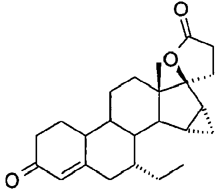 | 33 | 1,74 | 1,2 | 74 | 5,6 | 0,7 | 56,7 | 1,45 |
| 5 | 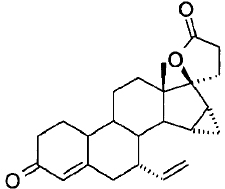 | 15 | 1,16 | 1,4 | 55 | 3,0 | 3,4 | 66,1 | 0,83 |
| 6 | 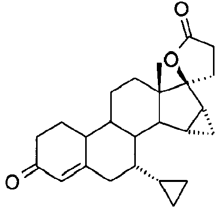 | 51 | 2,65 | 1,6 | 78 | 6,3 | 1,0 | 48,6 | 1,66 |
| 7 | 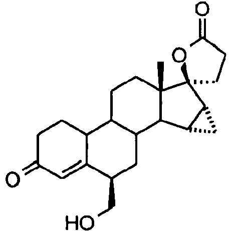 | 1100 | 49,90 | 2,1 | 10000 | 1000,0 | 910,0 | 22,8 | 23,76 |
| 8 | 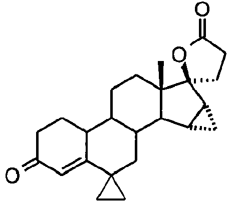 | 8,9 | 0,71 | 0,9 | 26 | 1,4 | 0,1 | 69,7 | 0,79 |
| 9 | 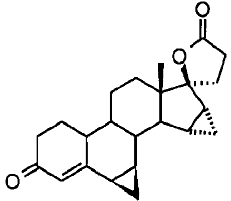 | 16 | 0,86 | 0,7 | 420 | 38,5 | 1 | 77,4 | 1,16 |
| 9 | 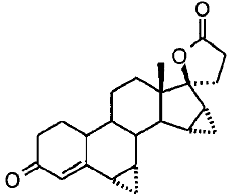 | 24 | 1,28 | 0,5 | 330 | 30,3 | 23 | 107,4 | 2,46 |

Die Verbindungen der Beispiele 1, 3-6 und 8-9 besitzen eine verbesserte Selektivität am Progesteronrezeptor (PR) verglichen mit dem Mineralokortikoidrezeptor (MR) ausgedrückt durch den Quotienten der Kompetitionsfaktoren KF-PR/KF-MR. Die Quotienten liegen in einem Bereich von 0,41-2,46 und damit deutlich unter dem von DRSP (5,4

## Patentansprüche

1. 15α,16α-Methylen-17-hydroxy-19-nor-17-pregna-4-en-3-on-21-carbonsäure γ-Lacton-Derivate ausgewählt aus der Gruppe:
17β-Hydroxy-15α,16α-methylen-19-nor-17α-pregna-4-en-3-on-21-carbonsäure γ-Lacton,
17β-Hydroxy-7α-methyl-15α,16α-methylen-19-nor-17α-pregna-4-en-3-on-21-carbonsäure γ-Lacton,
17β-Hydroxy-7β-methyl-15α,16α-methylen-19-nor-17α-pregna-4-en-3-on-21-carbonsäure γ-Lacton,
17β-Hydroxy-7α-ethyl-15α,16α-methylen-19-nor-17α-pregna-4-en-3-on-21-carbonsäure γ-Lacton,
17β-Hydroxy-7β-ethyl-15α,16α-methylen-19-nor-17α-pregna-4-en-3-on-21-carbonsäure γ-Lacton,
17β-Hydroxy-7α-vinyl-15α,16α-methylen-19-nor-17α-pregna-4-en-3-on-21-carbonsäure γ-Lacton,
17β-Hydroxy-7β-vinyl-15α,16α-methylen-19-nor-17α-pregna-4-en-3-on-21-carbonsäure γ-Lacton,
17β-Hydroxy-7α-cyclopropyl-15α,16α-methylen-19-nor-17α-pregna-4-en-3-on-21-carbonsäure γ-Lacton,
17β-Hydroxy-7β-cyclopropyl-15α,16α-methylen-19-nor-17α-pregna-4-en-3-on-21-carbonsäure γ-Lacton,
17β-Hydroxy-6-methylen-15α,16α-methylen-19-nor-17α-pregna-4-en-3-on-21-carbonsäure γ-Lacton,
17β-Hydroxy-6α-hydroxymethyl-15α,16α-methylen-19-nor-17α-pregna-4-en-3-on-21-carbonsäure γ-Lacton,
17β-Hydroxy-6α,7α,15α,16α-bismethylen-19-nor-17α-pregna-4-en-3-on-21-carbonsäure γ-Lacton,
17β-Hydroxy-6β,7β,15α,16α-bismethylen-19-nor-17α-pregna-4-en-3-on-21-carbonsäure γ-Lacton,
17β-Hydroxy-15α,16α-methylen-19-nor-17α-pregna-4,6-dien-3-on-21-carbonsäure γ-Lacton,
(E/Z)-3-(Hydroxyimino)-17β-hydroxy-15α,16α-methylen-19-nor-17α-pregna-4-en-21-carbonsäure γ-Lacton,
(E/Z)-3-(Hydroxyimino)-17β-hydroxy-7α-methyl-15α,16α-methylen-19-nor-17α-pregna-4-en-21-carbonsäure γ-Lacton,
(E/Z)-3-(Hydroxyimino)-17β-hydroxy-7β-methyl-15α,16α-methylen-19-nor-17α-pregna-4-en-21-carbonsäure γ-Lacton,
(E/Z)-3-(Hydroxyimino)-17β-hydroxy-7α-ethyl-15α,16α-methylen-19-nor-17α-pregna-4-en-21-carbonsäure γ-Lacton,
(E/Z)-3-(Hydroxyimino)-17β-hydroxy-7β-ethyl-15α,16α-methylen-19-nor-17α-pregna-4-en-21-carbonsäure γ-Lacton,
(E/Z)-3-(Hydroxyimino)-17β-hydroxy-7α-vinyl-15α,16α-methylen-19-nor-17α-pregna-4-en-21-carbonsäure γ-Lacton,
(E/Z)-3-(Hydroxyimino)-17β-hydroxy-7β-vinyl-15α,16α-methylen-19-nor-17α-pregna-4-en-21-carbonsäure γ-Lacton,
(E/Z)-3-(Hydroxyimino)-17β-hydroxy-7α-cyclopropyl-15α,16α-methylen-19-nor-17α-pregna-4-en-21-carbonsäure γ-Lacton,
(E/Z)-3-(Hydroxyimino)-17β-hydroxy-7β-cyclopropyl-15α,16α-methylen-19-nor-17α-pregna-4-en-21-carbonsäure γ-Lacton,
(E/Z)-3-(Hydroxyimino)-17β-hydroxy-6-methylen-15α,16α-methylen-19-nor-17α-pregna-4-en-21-carbonsäure γ-Lacton,
(E/Z)-3-(Hydroxyimino)-17β-hydroxy-6α-hydroxymethyl-15α,16α-methylen-19-nor-17α-pregna-4-en-21-carbonsäure γ-Lacton,
(E/Z)-3-(Hydroxyimino)-17β-hydroxy-6β-hydroxymethyl-15α,16α-methylen-19-nor-17α-pregna-4-en-21-carbonsäure γ-Lacton,
(E/Z)-3-(Hydroxyimino)-6,6-(1,2-Ethandiyl)-17β-hydroxy-15α,16α-methylen-19-nor-17α-pregna-4-en-21-carbonsäure γ-Lacton,
(E/Z)-3-(Hydroxyimino)-17β-hydroxy-6α,7α,15α,16α-bismethylen-19-nor-17α-pregna-4-en-21-carbonsäure γ-Lacton,
(E/Z)-3-(Hydroxyimino)-17β-hydroxy-6β,7β,15α,16α-bismethylen-19-nor-17α-pregna-4-en-21-carbonsäure γ-Lacton,
(E/Z)-3-(Hydroxyimino)-17β-hydroxy-15α,16α-methylen-19-nor-17α-pregna-4,6-dien-21-carbonsäure γ-Lacton,
17β-Hydroxy-18-methyl-15α,16α-methylen-19-nor-17α-pregna-4-en-3-on-21-carbonsäure γ-Lacton,
17β-Hydroxy-7α-methyl-18-methyl-15α,16α-methylen-19-nor-17α-pregna-4-en-3-on-21-carbonsäure γ-Lacton,
17β-Hydroxy-7β-methyl-18-methyl-15α,16α-methylen-19-nor-17α-pregna-4-en-3-on-21-carbonsäure γ-Lacton,
17β-Hydroxy-7α-ethyl-18-methyl-15α,16α-methylen-19-nor-17α-pregna-4-en-3-on-21-carbonsäure γ-Lacton,
17β-Hydroxy-7β-ethyl-18-methyl-15α,16α-methylen-19-nor-17α-pregna-4-en-3-on-21-carbonsäure γ-Lacton,
17β-Hydroxy-7α-vinyl-18-methyl-15α,16α-methylen-19-nor-17α-pregna-4-en-3-on-21-carbonsäure γ-Lacton,
17β-Hydroxy-7β-vinyl-18-methyl-15α,16α-methylen-19-nor-17α-pregna-4-en-3-on-21-carbonsäure γ-Lacton,
17β-Hydroxy-7α-cyclopropyl-18-methyl-15α,16α-methylen-19-nor-17α-pregna-4-en-3-on-21-carbonsäure γ-Lacton,
17β-Hydroxy-7β-cyclopropyl-18-methyl-15α,16α-methylen-19-nor-17α-pregna-4-en-3-on-21-carbonsäure γ-Lacton,
17β-Hydroxy-6-methylen-18-methyl-15α,16α-methylen-19-nor-17α-pregna-4-en-3-on-21-carbonsäure γ-Lacton,
17β-Hydroxy-6α-hydroxymethyl-18-methyl-15α,16α-methylen-19-nor-17α-pregna-4-en-3-on-21-carbonsäure γ-Lacton,
17β-Hydroxy-6β-hydroxymethyl-18-methyl-15α,16α-methylen-19-nor-17α-pregna-4-en-3-on-21-carbonsäure γ-Lacton,
17β-Hydroxy-6α,7α,15α,16α-bismethylen-19-nor-17α-pregna-4-en-3-on-21-carbonsäure γ-Lacton,
17β-Hydroxy-6β,7β,15α,16α-bismethylen-19-nor-17α-pregna-4-en-3-on-21-carbonsäure γ-Lacton,
17β-Hydroxy-18-methyl-15α,16α-methylen-19-nor-17α-pregna-4,6-dien-3-on-21-carbonsäure γ-Lacton,
(E/Z)-3-(Hydroxyimino)-17β-hydroxy-18-methyl-15α,16α-methylen-19-nor-17α-pregna-4-en-21-carbonsäure γ-Lacton,
(E/Z)-3-(Hydroxyimino)-17β-hydroxy-7α-methyl-18-methyl-15α,16α-methylen-19-nor-17α-pregna-4-en-21-carbonsäure γ-Lacton,
(E/Z)-3-(Hydroxyimino)-17β-hydroxy-7β-methyl-18-methyl-15α,16α-methylen-19-nor-17α-pregna-4-en-21-carbonsäure γ-Lacton,
(E/Z)-3-(Hydroxyimino)-17β-hydroxy-7α-ethyl-18-methyl-15α,16α-methylen-19-nor-17α-pregna-4-en-21-carbonsäure γ-Lacton,
(E/Z)-3-(Hydroxyimino)-17β-hydroxy-7β-ethyl-18-methyl-15α,16α-methylen-19-nor-17α-pregna-4-en-21-carbonsäure γ-Lacton,
(E/Z)-3-(Hydroxyimino)-17β-hydroxy-7α-vinyl-18-methyl-15α,16α-methylen-19-nor-17α-pregna-4-en-21-carbonsäure γ-Lacton,
(E/Z)-3-(Hydroxyimino)-17β-hydroxy-7β-vinyl-18-methyl-15α,16α-methylen-19-nor-17α-pregna-4-en-21-carbonsäure γ-Lacton,
(E/Z)-3-(Hydroxyimino)-17β-hydroxy-7α-cyclopropyl-18-methyl-15α,16α-methylen-19-nor-17α-pregna-4-en-21-carbonsäure γ-Lacton, (E/Z)-3-(Hydroxyimino)-17β-hydroxy-7β-cyclopropyl-18-methyl-15α,16α-methylen-19-nor-17α-pregna-4-en-21-carbonsäure γ-Lacton,
(E/Z)-3-(Hydroxyimino)-17β-hydroxy-6-methylen-18-methyl-15α,16α-methylen-19-nor-17α-pregna-4-en-21-carbonsäure γ-Lacton,
(E/Z)-3-(Hydroxyimino)-17β-hydroxy-6α-hydroxymethyl-18-methyl-15α,16α-methylen-19-nor-17α-pregna-4-en-21-carbonsäure γ-Lacton,
(E/Z)-3-(Hydroxyimino)-17β-hydroxy-6β-hydroxymethyl-18-methyl-15α,16α-methylen-19-nor-17α-pregna-4-en-21-carbonsäure γ-Lacton,
(E/Z)-3-(Hydroxyimino)-6,6-(1,2-Ethandiyl)-17β-hydroxy-18-methyl-15α,16α-methylen-19-nor-17α-pregna-4-en-21-carbonsäure γ-Lacton,
(E/Z)-3-(Hydroxyimino)-17β-hydroxy-6α,7α,15α,16α-bismethylen-19-nor-17α-pregna-4-en-21-carbonsäure γ-Lacton,
(E/Z)-3-(Hydroxyimino)-17β-hydroxy-6β,7β,15α,16α-bismethylen-19-nor-17α-pregna-4-en-21-carbonsäure γ-Lacton,
(E/Z)-3-(Hydroxyimino)-17β-hydroxy-18-methyl-15α,16α-methylen-19-nor-17α-pregna-4,6-dien-21-carbonsäure γ-Lacton.

2. 15α,16α-Methylen-17-hydroxy-19-nor-17-pregna-4-en-3-on-21-carbonsäure γ-Lacton-Derivate nach Anspruch 1 zur oralen Kontrazeption und zur Behandlung von prä-, peri- und postmenopausalen Beschwerden.

3. Verwendung der 15α,16α-Methylen-17-hydroxy-19-nor-17-pregna-4-en-3-on-21-carbonsäure γ-Lacton-Derivate nach Anspruch 1 zur Herstellung eines Arzneimittels zur oralen Kontrazeption und zur Behandlung von prä-, peri- und postmenopausalen Beschwerden.

4. Verwendung nach Anspruch 3, **dadurch gekennzeichnet, daß** das Arzneimittel gestagene, antimineralkortikoide und androgene Wirkung aufweist.

5. Arzneimittel enthaltend mindestens ein 15α,16α-Methylen-17-hydroxy-19-nor-17-pregna-4-en-3-on-21-carbonsäure γ-Lacton-Derivat nach Anspruch 1 sowie mindestens einen geeigneten pharmazeutisch unbedenklichen Zusatzstoff.

6. Arzneimittel nach Anspruch 5, enthaltend außerdem mindestens ein Estrogen.

7. Arzneimittel nach Anspruch 6, **dadurch gekennzeichnet, daß** das Estrogen Ethinylestradiol ist.

8. Arzneimittel nach Anspruch 6, **dadurch gekennzeichnet, daß** das Estrogen ein natürliches Estrogen ist.

9. Arzneimittel nach Anspruch 8, **dadurch gekennzeichnet, daß** das natürliche Estrogen Estradiol ist.

10. Arzneimittel nach Anspruch 8, **dadurch gekennzeichnet, daß** das natürliche Estrogen Estradiolvalerat ist.

11. Arzneimittel nach Anspruch 8, **dadurch gekennzeichnet, daß** das natürliche Estrogen ein konjugiertes Estrogen ist.

12. Verwendung eines 15α,16α-Methylen-17-hydroxy-19-nor-17-pregna-4-en-3-on-21-carbonsäure γ-Lacton-Derivates nach Anspruch 1 zur Herstellung eines Arzneimittels zur intrauterinen Anwendung.

13. Verwendung nach Anspruch 12 zur Herstellung eines intrauterinen Systems (I-US).

14. Arzneimittel nach einem der Ansprüche 5 - 11, **dadurch gekennzeichnet, dass** es zur intrauterinen Anwendung hergerichtet ist.

15. Arzneimittel nach Anspruch 14, **dadurch gekennzeichnet, dass** es ein intrauterines System ist.

## Claims

1. 15α,16α-Methylene-17-hydroxy-19-nor-17-pregna-4-en-3-one-21-carboxylic acid γ-lactone derivatives selected from the group
17β-Hydroxy-15α,16α-methylene-19-nor-17α-pregna-4-en-3-one-21-carboxylic acid γ-lactone
17β-Hydroxy-7α-methyl-15α,16α-methylene-19-nor-17α-pregna-4-en-3-one-21-carboxylic acid γ-lactone
17β-Hydroxy-7β-methyl-15α,16α-methylene-19-nor-17α-pregna-4-en-3-one-21-carboxylic acid γ-lactone
17β-Hydroxy-7α-ethyl-15α,16α-methylene-19-nor-17α-pregna-4-en-3-one-21-carboxylic acid γ-lactone
17β-Hydroxy-7β-ethyl-15α,16α-methylene-19-nor-17α-pregna-4-en-3-one-21-carboxylic acid γ-lactone
17β-Hydroxy-7α-vinyl-15α,16α-methylene-19-nor-17α-pregna-4-en-3-one-21-carboxylic acid γ-lactone
17β-Hydroxy-7β-vinyl-15α,16α-methylene-19-nor-17α-pregna-4-en-3-one-21-carboxylic acid γ-lactone
17β-Hydroxy-7α-cyclopropyl-15α,16α-methylene-19-nor-17α-pregna-4-en-3-one-21-carboxylic acid γ-lactone
17β-Hydroxy-7β-cyclopropyl-15α,16α-methylene-19-nor-17α-pregna-4-en-3-one-21-carboxylic acid γ-lactone
17β-Hydroxy-6-methylene-15α,16α-methylene-19-nor-17α-pregna-4-en-3-one-21-carboxylic acid γ-lactone
17β-Hydroxy-6α-hydroxymethyl-15α,16α-methylene-19-nor-17α-pregna-4-en-3-one-21-carboxylic acid γ-lactone
17β-Hydroxy-6α,7α,15α,16α-bismethylene-19-nor-17α-pregna-4-en-3-one-21-carboxylic acid γ-lactone
17β-Hydroxy-6β,7β,15α,16α-bismethylene-19-nor-17α-pregna-4-en-3-one-21-carboxylic acid γ-lactone
17β-Hydroxy-15α,16α-methylene-19-nor-17α-pregna-4,6-dien-3-one-21-carboxylic acid γ-lactone
(E/Z)-3-(Hydroxyimino)-17β-hydroxy-15α,16α-methylene-19-nor-17α-pregna-4-ene-21-carboxylic acid γ-lactone
(E/Z)-3-(Hydroxyimino)-17β-hydroxy-7α-methyl-15α,16α-methylene-19-nor-17α-pregna-4-ene-21-carboxylic acid γ-lactone
(E/Z)-3-(Hydroxyimino)-17β-hydroxy-7β-methyl-15α,16α-methylene-19-nor-17α-pregna-4-ene-21-carboxylic acid γ-lactone
(E/Z)-3-(Hydroxyimino)-17β-hydroxy-7α-ethyl-15α,16α-methylene-19-nor-17α-pregna-4-ene-21-carboxylic acid γ-lactone
(E/Z)-3-(Hydroxyimino)-17β-hydroxy-7β-ethyl-15α,16α-methylene-19-nor-17α-pregna-4-ene-21-carboxylic acid γ-lactone
(E/Z)-3-(Hydroxyimino)-17β-hydroxy-7α-vinyl-15α,16α-methylene-19-nor-17α-pregna-4-ene-21-carboxylic acid γ-lactone
(E/Z)-3-(Hydroxyimino)-17β-hydroxy-7β-vinyl-15α,16α-methylene-19-nor-17α-pregna-4-ene-21-carboxylic acid γ-lactone
(E/Z)-3-(Hydroxyimino)-17β-hydroxy-7α-cyclopropyl-15α,16α-methylene-19-nor-17α-pregna-4-ene-21-carboxylic acid γ-lactone
(E/Z)-3-(Hydroxyimino)-17β-hydroxy-7β-cyclopropyl-15α,16α-methylene-19-nor-17α-pregna-4-ene-21-carboxylic acid γ-lactone
(E/Z)-3-(Hydroxyimino)-17β-hydroxy-6-methylene-15α,16α-methylene-19-nor-17α-pregna-4-ene-21-carboxylic acid γ-lactone
(E/Z)-3-(Hydroxyimino)-17β-hydroxy-6α-hydroxymethyl-15α,16α-methylene-19-nor-17α-pregna-4-ene-21-carboxylic acid γ-lactone
(E/Z)-3-(Hydroxyimino)-17β-hydroxy-6β-hydroxymethyl-15α,16α-methylene-19-nor-17α-pregna-4-ene-21-carboxylic acid γ-lactone
(E/Z)-3-(Hydroxyimino)-6,6-(1,2-ethanediyl)-17β-hydroxy-15α,16α-methylene-19-nor-17α-pregna-4-ene-21-carboxylic acid γ-lactone
(E/Z)-3-(Hydroxyimino)-17β-hydroxy-6α,7α,15α,16α-bismethylene-19-nor-17a-pregna-4-ene-21-carboxylic acid γ-lactone
(E/Z)-3-(Hydroxyimino)-17β-hydroxy-6β,7β,15α,16α-bismethylene-19-nor-17a-pregna-4-ene-21-carboxylic acid γ-lactone
(E/Z)-3-(Hydroxyimino)-17β-hydroxy-15α,16α-methylene-19-nor-17α-pregna-4,6-diene-21-carboxylic acid γ-lactone
17β-Hydroxy-18-methyl-15α,16α-methylene-19-nor-17α-pregna-4-en-3-one-21-carboxylic acid γ-lactone
17β-Hydroxy-7α-methyl-18-methyl-15α,16α-methylene-19-nor-17α-pregna-4-en-3-one-21-carboxylic acid γ-lactone
17β-Hydroxy-7β-methyl-18-methyl-15α,16α-methylene-19-nor-17α-pregna-4-en-3-one-21-carboxylic acid γ-lactone
17β-Hydroxy-7α-ethyl-18-methyl-15α,16α-methylene-19-nor-17α-pregna-4-en-3-one-21-carboxylic acid γ-lactone
17β-Hydroxy-7β-ethyl-18-methyl-15α,16α-methylene-19-nor-17α-pregna-4-en-3-one-21-carboxylic acid γ-lactone
17β-Hydroxy-7α-vinyl-18-methyl-15α,16α-methylene-19-nor-17α-pregna-4-en-3-one-21-carboxylic acid γ-lactone
17β-Hydroxy-7β-vinyl-18-methyl-15α,16α-methylene-19-nor-17α-pregna-4-en-3-one-21-carboxylic acid γ-lactone
17β-Hydroxy-7α-cyclopropyl-18-methyl-15α,16α-methylene-19-nor-17α-pregna-4-en-3-one-21-carboxylic acid γ-lactone
17β-Hydroxy-7β-cyclopropyl-18-methyl-15α,16α-methylene-19-nor-17α-pregna-4-en-3-one-21-carboxylic acid γ-lactone
17β-Hydroxy-6-methylene-18-methyl-15α,16α-methylene-19-nor-17α-pregna-4-en-3-one-21-carboxylic acid γ-lactone
17β-Hydroxy-6α-hydroxymethyl-18-methyl-15α,16α-methylene-19-nor-17α-pregna-4-en-3-one-21-carboxylic acid γ-lactone
17β-Hydroxy-6β-hydroxymethyl-18-methyl-15α,16α-methylene-19-nor-17α-pregna-4-en-3-one-21-carboxylic acid γ-lactone
17β-Hydroxy-6α,7α,15α,16α-bismethylene-19-nor-17α-pregna-4-en-3-one-21-carboxylic acid γ-lactone
17β-Hydroxy-6β,7β,15α,16α-bismethylene-19-nor-17α-pregna-4-en-3-one-21-carboxylic acid γ-lactone
17β-Hydroxy-18-methyl-15α,16α-methylene-19-nor-17α-pregna-4,6-dien-3-one-21-carboxylic acid γ-lactone
(E/Z)-3-(Hydroxyimino)-17β-hydroxy-18-methyl-15α,16α-methylene-19-nor-17α-pregna-4-ene-21-carboxylic acid γ-lactone
(E/Z)-3-(Hydroxyimino)-17β-hydroxy-7α-methyl-18-methyl-15α,16α-methylene-19-nor-17α-pregna-4-ene-21-carboxylic acid γ-lactone
(E/Z)-3-(Hydroxyimino)-17β-hydroxy-7β-methyl-18-methyl-15α,16α-methylene-19-nor-17α-pregna-4-ene-21-carboxylic acid γ-lactone
(E/Z)-3-(Hydroxyimino)-17β-hydroxy-7α-ethyl-18-methyl-15α,16α-methylene-19-nor-17α-pregna-4-ene-21-carboxylic acid γ-lactone
(E/Z)-3-(Hydroxyimino)-17β-hydroxy-7β-ethyl-18-methyl-15α,16α-methylene-19-nor-17α-pregna-4-ene-21-carboxylic acid γ-lactone
(E/Z)-3-(Hydroxyimino)-17β-hydroxy-7α-vinyl-18-methyl-15α,16α-methylene-19-nor-17α-pregna-4-ene-21-carboxylic acid γ-lactone
(E/Z)-3-(Hydroxyimino)-17β-hydroxy-7β-vinyl-18-methyl-15α,16α-methylene-19-nor-17α-pregna-4-ene-21-carboxylic acid γ-lactone
(E/Z)-3-(Hydroxyimino)-17β-hydroxy-7α-cyclopropyl-18-methyl-15α,16α-methylene-19-nor-17α-pregna-4-ene-21-carboxylic acid γ-lactone
(E/Z)-3-(Hydroxyimino)-17β-hydroxy-7β-cyclopropyl-18-methyl-15α,16α-methylene-19-nor-17α-pregna-4-ene-21-carboxylic acid γ-lactone
(E/Z)-3-(Hydroxyimino)-17β-hydroxy-6-methylene-18-methyl-15α,16α-methylene-19-nor-17α-pregna-4-ene-21-carboxylic acid γ-lactone
(E/Z)-3-(Hydroxyimino)-17β-hydroxy-6α-hydroxymethyl-18-methyl-15α,16α-methylene-19-nor-17α-pregna-4-ene-21-carboxylic acid γ-lactone
(E/Z)-3-(Hydroxyimino)-17β-hydroxy-6β-hydroxymethyl-18-methyl-15α,16α-methylene-19-nor-17α-pregna-4-ene-21-carboxylic acid γ-lactone
(E/Z)-3-(Hydroxyimino)-6,6-(1,2-ethanediyl)-17β-hydroxy-18-methyl-15α,16α-methylene-19-nor-17α-pregna-4-ene-21-carboxylic acid γ-lactone
(E/Z)-3-(Hydroxyimino)-17β-hydroxy-6α,7α,15α,16α-bismethylene-19-nor-17a-pregna-4-ene-21-carboxylic acid γ-lactone
(E/Z)-3-(Hydroxyimino)-17β-hydroxy-6β,7β,15α,16α-bismethylene-19-nor-17a-pregna-4-ene-21-carboxylic acid γ-lactone
(E/Z)-3-(Hydroxyimino)-17β-hydroxy-18-methyl-15α,16α-methylene-19-nor-17α-pregna-4,6-diene-21-carboxylic acid γ-lactone.

2. 15α,16α-Methylene-17-hydroxy-19-nor-17-pregna-4-en-3-one-21-carboxylic acid γ-lactone derivatives as claimed in claim 1 for oral contraception and for the treatment of pre-, peri- and postmenopausal complaints.

3. The use of the 15α,16α-methylene-17-hydroxy-19-nor-17-pregna-4-en-3-one-21-carboxylic acid γ-lactone derivatives as claimed in claim 1 for the production of a medicinal product for oral contraception and for the treatment of pre-, peri- and postmenopausal complaints.

4. The use as claimed in claim 3, **characterized in that** the medicinal product has progestational, antimineralocorticoid and androgenic action.

5. A medicinal product containing at least one 15α,16α-methylene-17-hydroxy-19-nor-17-pregna-4-en-3-one-21-carboxylic acid γ-lactone derivative as claimed in claim 1 and at least one suitable pharmaceutically harmless additive.

6. The medicinal product as claimed in claim 5, additionally containing at least one estrogen.

7. The medicinal product as claimed in claim 6, **characterized in that** the estrogen is ethinylestradiol.

8. The medicinal product as claimed in claim 6, **characterized in that** the estrogen is a natural estrogen.

9. The medicinal product as claimed in claim 8, **characterized in that** the natural estrogen is estradiol.

10. The medicinal product as claimed in claim 8, **characterized in that** the natural estrogen is estradiolvalerate.

11. The medicinal product as claimed in claim 8, **characterized in that** the natural estrogen is a conjugated estrogen.

12. The use of a 15α,16α-methylene-17-hydroxy-19-nor-17-pregna-4-en-3-one-21-carboxylic acid γ-lactone derivative as claimed in claim 1 for the production of a medicinal product for intrauterine use.

13. The use as claimed in claim 12 for the production of an intrauterine system (IUS).

14. The medicinal product as claimed in any of claims 5-11, **characterized in that** it is designed for intrauterine use.

15. The medicinal product as claimed in claim 14, **characterized in that** it is an intrauterine system.

## Revendications

1. Dérivés de γ-lactone d'acide 15α,16α-méthylène-17-hydroxy-19-nor-17-prégna-4-én-3-one-21-carboxylique choisis dans le groupe :
γ-lactone d'acide 17β-hydroxy-15α,16α-méthylène-19-nor-17α-prégna-4-én-3-one-21-carboxylique,
γ-lactone d'acide 17β-hydroxy-7α-méthyl-15α,16α-méthylène-19-nor-17α-prégna-4-én-3-one-21-carboxylique,
γ-lactone d'acide 17β-hydroxy-7β-méthyl-15α,16α-méthylène-19-nor-17α-prégna-4-én-3-one-21-carboxylique,
γ-lactone d'acide 17β-hydroxy-7α-éthyl-15α,16α-méthylène-19-nor-17α-prégna-4-én-3-one-21-carboxylique,
γ-lactone d'acide 17β-hydroxy-7β-éthyl-15α,16α-méthylène-19-nor-17α-prégna-4-én-3-one-21-carboxylique,
γ-lactone d'acide 17β-hydroxy-7α-vinyl-15α,16α-méthylène-19-nor-17α-prégna-4-én-3-one-21-carboxylique,
γ-lactone d'acide 17β-hydroxy-7β-vinyl-15α,16α-méthylène-19-nor-17α-prégna-4-én-3-on-21-carboxylique,
γ-lactone d'acide 17β-hydroxy-7α-cyclopropyl-15α,16α-méthylène-19-nor-17α-prégna-4-én-3-one-21-carboxylique,
γ-lactone d'acide 17β-hydroxy-7β-cyclopropyl-15α,16α-méthylène-19-nor-17α-prégna-4-én-3-one-21-carboxylique,
γ-lactone d'acide 17β-hydroxy-6-méthylène-15α,16α-méthylène-19-nor-17α-prégna-4-én-3-one-21-carboxylique,
γ-lactone d'acide 17β-hydroxy-6α-hydroxyméthyl-15α,16α-méthylène-19-nor-17α-prégna-4-én-3-one-21-carboxylique,
γ-lactone d'acide 17β-hydroxy-6α,7α,15α,16α-bis-méthylène-19-nor-17α-prégna-4-én-3-one-21-carboxylique,
γ-lactone d'acide 17β-hydroxy-6β,7β,15α,16α-bis-méthylène-19-nor-17α-prégna-4-én-3-one-21-carboxylique,
γ-lactone d'acide 17β-hydroxy-15α,16α-méthylène-19-nor-17α-prégna-4,6-dién-3-one-21-carboxylique,
γ-lactone d'acide (E/Z)-3-(hydroxyimino)-17β-hydroxy-15α,16α-méthylène-19-nor-17α-prégna-4-ène-21-carboxylique,
γ-lactone d'acide (E/Z)-3-(hydroxyimino)-17β-hydroxy-7α-méthyl-15α,16α-méthylène-19-nor-17α-prégna-4-ène-21-carboxylique,
γ-lactone d'acide (E/Z)-3-(hydroxyimino)-17β-hydroxy-7β-méthyl-15α,16α-méthylène-19-nor-17α-prégna-4-ène-21-carboxylique,
γ-lactone d'acide (E/Z)-3-(hydroxyimino)-17β-hydroxy-7α-éthyl-15α,16α-méthylène-19-nor-17α-prégna-4-ène-21-carboxylique,
γ-lactone d'acide (E/Z)-3-(hydroxyimino)-17β-hydroxy-7β-éthyl-15α,16α-méthylène-19-nor-17α-prégna-4-ène-21-carboxylique,
γ-lactone d'acide (E/Z)-3-(hydroxyimino)-17β-hydroxy-7α-vinyl-15α,16α-méthylène-19-nor-17α-prégna-4-ène-21-carboxylique,
γ-lactone d'acide (E/Z)-3-(hydroxyimino)-17β-hydroxy-7β-vinyl-15α,16α-méthylène-19-nor-17α-prégna-4-ène-21-carboxylique,
γ-lactone d'acide (E/Z)-3-(hydroxyimino)-17β-hydroxy-7α-cyclopropyl-15α,16α-méthylène-19-nor-17α-prégna-4-ène-21-carboxylique
γ-lactone d'acide (E/Z)-3-(hydroxyimino)-17β-hydroxy-7β-cyclopropyl-15α,16α-méthylène-19-nor-17α-prégna-4-ène-21-carboxylique,
γ-lactone d'acide (E/Z)-3-(hydroxyimino)-17β-hydroxy-6-méthylène-15α,16α-méthylène-19-nor-17α-prégna-4-ène-21-carboxylique,
γ-lactone d'acide (E/Z)-3-(hydroxyimino)-17β-hydroxy-6α-hydroxyméthyl-15α,16α-méthylène-19-nor-17α-prégna-4-ène-21-carboxylique,
γ-lactone d'acide (E/Z)-3-(hydroxyimino)-17β-hydroxy-6β-hydroxyméthyl-15α,16α-méthylène-19-nor-17α-prégna-4-ène-21-carboxylique,
γ-lactone d'acide (E/2)-3-(hydroxyimino)-6,6-(1,2-éthanediyl)-17β-hydroxy-15α,16α-méthylène-19-nor-17α-prégna-4-ène-21-carboxylique,
γ-lactone d'acide (E/Z)-3-(hydroxyimino)-17β-hydroxy-6α,7α,15α,16α-bisméthylène-19-nor-17α-prégna-4-ène-21-carboxylique,
γ-lactone d'acide (E/Z)-3-(hydroxyimino)-17β-hydroxy-6β,7β,15α,16α-bisméthylène-19-nor-17α-prégna-4-ène-21-carboxylique,
γ-lactone d'acide (E/Z)-3-(hydroxyimino)-17β-hydroxy-15α,16α-méthylène-19-nor-17α-prégna-4,6-diène-21-carboxylique,
γ-lactone d'acide 17β-hydroxy-18-méthyl-15α,16α-méthylène-19-nor-17α-prégna-4-én-3-one-21-carboxylique,
γ-lactone d'acide 17β-hydroxy-7α-méthyl-18-méthyl-15α,16α-méthylène-19-nor-17α-prégna-4-én-3-one-21-carboxylique,
γ-lactone d'acide 17β-hydroxy-7β-méthyl-18-méthyl-15α,16α-méthylène-19-nor-17α-prégna-4-én-3-one-21-carboxylique,
γ-lactone d'acide 17β-hydroxy-7α-éthyl-18-méthyl-15α,16α-méthylène-19-nor-17α-prégna-4-én-3-one-21-carboxylique,
γ-lactone d'acide 17β-hydroxy-7β-éthyl-18-méthyl-15a,16a-méthylène-19-nor-17α-prégna-4-én-3-one-21-carboxylique,
γ-lactone d'acide 17β-hydroxy-7α-vinyl-18-méthyl-15α,16α-méthylène-19-nor-17α-prégna-4-én-3-one-21-carboxylique,
γ-lactone d'acide 17β-hydroxy-7β-vinyl-18-méthyl-15α,16α-méthylène-19-nor-17α-prégna-4-én-3-one-21-carboxylique,
γ-lactone d'acide 17β-hydroxy-7α-cyclopropyl-18-méthyl-15α,16α-méthylène-19-nor-17α-prégna-4-én-3-one-21-carboxylique,
γ-lactone d'acide 17β-hydroxy-7β-cyclopropyl-18-méthyl-15α,16α-méthylène-19-nor-17α-prégna-4-én-3-one-21-carboxylique,
γ-lactone d'acide 17β-hydroxy-6-méthylène-18-méthyl-15α,16α-méthylène-19-nor-17α-prégna-4-én-3-one-21-carboxylique,
γ-lactone d'acide 17β-hydroxy-6α-hydroxyméthyl-18-méthyl-15α,16α-méthylène-19-nor-17α-prégna-4-én-3-one-21-carboxylique,
γ-lactone d'acide 17β-hydroxy-6β-hydroxyméthyl-18-méthyl-15α,16α-méthylène-19-nor-17α-prégna-4-én-3-one-21-carboxylique,
γ-lactone d'acide 17β-hydroxy-6α,7α,15α,16α-bis-méthylène-19-nor-17α-prégna-4-én-3-one-21-carboxylique,
γ-lactone d'acide 17β-hydroxy-6β,7β,15α,16α-bis-méthylène-19-nor-17α-prégna-4-én-3-one-21-carboxylique,
γ-lactone d'acide 17β-hydroxy-18-méthyl-15α,16α-méthylène-19-nor-17α-prégna-4,6-dién-3-one-21-carboxylique,
γ-lactone d'acide (E/Z)-3-(hydroxyimino)-17β-hydroxy-18-méthyl-15α,16α-méthylène-19-nor-17α-prégna-4-ène-21-carboxylique,
γ-lactone d'acide (E/Z)-3-(hydroxyimino)-17β-hydroxy-7α-méthyl-18-méthyl-15α,16α-méthylène-19-nor-17α-prégna-4-ène-21-carboxylique,
γ-lactone d'acide (E/Z)-3-(hydroxyimino)-17β-hydroxy-7β-méthyl-18-méthyl-15α,16α-méthylène-19-nor-17α-prégna-4-ène-21-carboxylique,
γ-lactone d'acide (E/Z)-3-(hydroxyimino)-17β-hydroxy-7α-éthyl-18-méthyl-15α,16α-méthylène-19-nor-17α-prégna-4-ène-21-carboxylique,
γ-lactone d'acide (E/Z)-3-(hydroxyimino)-17β-hydroxy-7β-éthyl-18-méthyl-15α,16α-méthylène-19-nor-17α-prégna-4-ène-21-carboxylique,
γ-lactone d'acide (E/Z)-3-(hydroxyimino)-17β-hydroxy-7α-vinyl-18-méthyl-15α,16α-méthylène-19-nor-17α-prégna-4-ène-21-carboxylique,
γ-lactone d'acide (E/Z)-3-(hydroxyimino)-17β-hydroxy-7β-vinyl-18-méthyl-15α,16α-méthylène-19-nor-17α-prégna-4-ène-21-carboxylique,
γ-lactone d'acide (E/Z)-3-(hydroxyimino)-17β-hydroxy-7α-cyclopropyl-18-méthyl-15α,16α-méthylène-19-nor-17α-prégna-4-ène-21-carboxylique,
γ-lactone d'acide (E/Z)-3-(hydroxyimino)-17β-hydroxy-7β-cyclopropyl-18-méthyl-15α,16α-méthylène-19-nor-17α-prégna-4-ène-21-carboxylique,
γ-lactone d'acide (E/Z)-3-(hydroxyimino)-17β-hydroxy-6-méthylène-18-méthyl-15α,16α-méthylène-19-nor-17α-prégna-4-ène-21-carboxylique,
γ-lactone d'acide (E/Z)-3-(hydroxyimino)-17β-hydroxy-6α-hydroxyméthyl-18-méthyl-15α,16α-méthylène-19-nor-17α-prégna-4-ène-21-carboxylique,
γ-lactone d'acide (E/Z)-3-(hydroxyimino)-17β-hydroxy-6β-hydroxyméthyl-18-méthyl-15α,16α-méthylène-19-nor-17α-prégna-4-ène-21-carboxylique,
γ-lactone d'acide (E/2)-3-(hydroxyimino)-6,6-(1,2-éthanediyl)-17β-hydroxy-18-méthyl-15α,16α-méthylène-19-nor-17α-prégna-4-ène-21-carboxylique,
γ-lactone d'acide (E/Z)-3-(hydroxyimino)-17β-hydroxy-6α,7α,15α,16α-bisméthylène-19-nor-17α-prégna-4-ène-21-carboxylique,
γ-lactone d'acide (E/Z)-3-(hydroxyimino)-17β-hydroxy-6β,7β,15α,16α-bisméthylène-19-nor-17α-prégna-4-ène-21-carboxylique,
γ-lactone d'acide (E/Z)-3-(hydroxyimino)-17β-hydroxy-18-méthyl-15α,16α-méthylène-19-nor-17α-prégna-4,6-diène-21-carboxylique.

2. Dérivés de γ-lactone d'acide 15α,16α-méthylène-17-hydroxy-19-nor-17-prégna-4-én-3-one-21-carboxylique selon la revendication 1, pour la contraception orale et pour le traitement de troubles pré-, péri- et post-ménopausiques.

3. Utilisation des dérivés de γ-lactone d'acide 15α,16α-méthylène-17-hydroxy-19-nor-17-prégna-4-én-3-one-21-carboxylique selon la revendication 1, pour la fabrication d'un médicament destiné à la contraception orale et au traitement de troubles pré-, péri- et post-ménopausiques.

4. Utilisation selon la revendication 3, **caractérisée en ce que** le médicament a une action progestative, antiminéralocorticoïde et androgène.

5. Médicament contenant au moins un dérivé de γ-lactone d'acide 15α,16α-méthylène-17-hydroxy-19-nor-17-prégna-4-én-3-one-21-carboxylique selon la revendication 1 ainsi qu'au moins un additif approprié, pharmaceutiquement acceptable.

6. Médicament selon la revendication 5, contenant en outre au moins un oestrogène.

7. Médicament selon la revendication 6, **caractérisé en ce que** l'oestrogène est l'éthinyl-estradiol.

8. Médicament selon la revendication 6, **caractérisé en ce que** l'oestrogène est un oestrogène naturel.

9. Médicament selon la revendication 8, **caractérisé en ce que** l'oestrogène naturel est l'estradiol.

10. Médicament selon la revendication 8, **caractérisé en ce que** l'oestrogène naturel est le valérate d'estradiol.

11. Médicament selon la revendication 8, **caractérisé en ce que** l'oestrogène naturel est un oestrogène conjugué.

12. Utilisation d'un dérivé de γ-lactone d'acide 15α,16α-méthylène-17-hydroxy-19-nor-17-prégna-4-én-3-one-21-carboxylique selon la revendication 1, pour la fabrication d'un médicament destiné à l'usage intra-utérin.

13. Utilisation selon la revendication 12, pour la fabrication d'un système intra-utérin (SIU).

14. Médicament selon l'une quelconque des revendications 5 - 11, **caractérisé en ce qu'**il est destiné à l'usage intra-utérin.

15. Médicament selon la revendication 14, **caractérisé en ce qu'**il est un système intra-utérin.
